# EUROPEAN PATENT APPLICATION

(11) **EP 3 828 290 A1**
(43) Date of publication of application: **02.06.2021**
(21) Application number: 18927940.9
(22) Date of filing: 26.07.2018
(51) Int. Cl.: C12Q 1/6886

(54) **METHOD FOR IDENTIFYING STATE OF BREAST CANCER AND KIT**

(71) Applicant: Excellen Medical Technology Co., Ltd., Science and Technology Park, Changpin District Beijing 102200 (CN)
(72) Inventor: LI, Mingming, Beijing 102200 (CN); XU, Chunye, Beijing 102200 (CN); LI, Shuyu, Beijing 102200 (CN); CHEN, Yanli, Beijing 102200 (CN); PU, Jue, Beijing 102200 (CN)
(74) Representative: Glück Kritzenberger Patentanwälte PartGmbB
(86) International application number: PCT/CN2018/097296
(87) International publication number: WO 2020/019268

(57) **Abstract**

Disclosed is a method for identifying the state of breast cancer status in human subjects subject, comprising: 1) collecting biological samples from the human subjects; 2) detecting methylation level of biomarker genes in the biological samples, wherein the biomarker genes are one or more selected from the following genes: APC, BRCA1, CCND2, CST6, GP5, GSTP1, PITX2, RARB, RASSF1A and SOX17; and 3) comparing the methylation level detected in step 2) with the normal methylation level of the corresponding biomarker gene in the colony to determine the state of breast cancer in human subjects. Further provided is a kit for identifying the state of breast cancerin human subjects.

## Description

### FIELD OF THE INVENTION

The present invention relates to a method and a kit for identifying a breast cancer status in a subject.

### BACKGROUND

Breast cancer is the most common malignant tumor in women. The probability of breast cancer in a woman's lifetime is about 10%. There are about 1.3 million breast cancer patients in the world each year, about 400,000 people die from the disease and the number is increased at a rate of 2% ∼3% per year. In the United States, the incidence of breast cancer occupies the first place among female malignant tumors, and its mortality ranks second in the mortality rate of malignant tumors. In our country, the incidence of breast cancer has also leapt to the first place among female malignant tumors, and the age of onset is showing a younger trend. Breast cancer has become the number one killer of women's health worldwide. The survival period of breast cancer patients is closely related to the pathological stage. The earlier the pathological stage, the higher the cure rate. Early diagnosis is particularly crucial for breast cancer patients. Multiple studies have shown that large-scale breast cancer screening is able to detect more early cases and gradually reduce mortality against a background of rising prevalence.

At present, breast cancer screening mainly relies on imaging methods, such as mammography, ultrasound imaging, and magnetic resonance imaging. However, early breast cancer often lacks typical symptoms and signs, which makes imaging technology unable to show good results in early breast cancer screening. With the gradual investigation of the molecular mechanism of breast cancer, the development of molecular markers for the early diagnosis of breast cancer is highly concerned. Unfortunately, no molecular marker with high sensitivity and specificity has been found so far. In recent years, research on breast cancer epigenetics has made rapid progress, especially in DNA methylation. It has been found that many specific tumor-related genes have different degrees of methylation status change in the early stage of breast cancer, which provides a new opportunity for the exploration of markers for early diagnosis of breast cancer.

Abnormal DNA methylation of the genome and the occurrence of tumors have always been one of the hotspots in medical research. All of cell cycle, DNA repair, angiogenesis, cell apoptosis and the like involve the methylation of related genes. The most likely regulatory role of DNA hypermethylation is the suppression of the expression of key genes, thereby determining the fate of a cell. For example, the study of abnormal DNA methylation in tumor cells has made many significant advances in various tumors. In mammals, methylation only affects a cytosine in front of a guanine (CpG) on a DNA strand. The methylation distribution of CpG dinucleotides in normal cells is not uniform. About 50% of genes have CpG islands with concentrated distribution of CpGs in the promoter region, with lengths ranging from 0.5 to 2 kb. This region is closely related to gene transcription regulation. In humans, the methylation of CpG islands in certain gene regulatory regions occurs frequently in relevant cancer cell tissues, showing a correlation with the onset, disease progression, prognosis, drug sensitivity, and the like of certain tumors. To date, gene methylation abnormalities have been found in most human tumors. Studies have found that epigenetic coding in cancer cells is disturbed, first of all manifested in the disturbance of DNA methylation level, also known as methylation rearrangement. Since the local hypermethylation of a CpG island in a tumor suppressor gene is earlier than the malignant proliferation of cells, the detection of DNA methylation can be used for the early diagnosis of tumorigenesis. Methylation of cancer-related genes is also an early event of breast cancer, so the methylation status of related genes has become an effective indicator for the risk prediction of early breast cancer. Even so, there is still a lack of means to effectively detect the methylation status of these cancer-related genes and process the detected results.

### SUMMARY

In order to solve the above problems, in one aspect, a method for identifying a breast cancer status in a subject is provided herein, which comprises the following steps: 1) collecting a biological sample from the subject; 2) detecting methylation level(s) of a biomarker gene in the biological sample, wherein the biomarker gene(s) is/are selected from one or more of the following genes: APC, BRCA1, CCND2, CST6, GP5, GSTP1, PITX2, RARB, RASSF1A and SOX17; and 3) comparing the methylation level(s) detected in step 2) with normal methylation level(s) of the corresponding biomarker gene(s) in a population to determine the breast cancer status in the subject.

In some embodiments, the method further comprises performing steps 1) and 2) again after the subject undergoes a medical treatment, and comparing the both obtained detection results of the methylation level(s) to determine change of the breast cancer status in the subject.

In some embodiments, step 2) can comprise extracting DNA from the biological sample and treating the extracted DNA with a bisulfite, so that unmethylated cytosine residues in the DNA are deaminated, and methylated cytosine residues remain unchanged.

In some preferred embodiments, the bisulfite is sodium bisulfite.

In some preferred embodiments, in step 2) the biomarker genes are selected from 2 or more of APC, BRCA1, CCND2, CST6, GP5, GSTP1, PITX2, RARB, RASSF1A and SOX17.

In more preferred embodiments, in step 2) the biomarker genes are selected from 5 or more of APC, BRCA1, CCND2, CST6, GP5, GSTP1, PITX2, RARB, RASSF1A and SOX17.

In some particular embodiments, in step 2) the biomarker genes are BRCA1, CCND2, PITX2, RARB and RASSF1A.

In some preferred embodiments, the breast cancer status is breast cancer stage I or stage II, and the biomarker gene(s) is/are APC and/or RASSF1A.

In some preferred embodiments, the breast cancer status is an noninfiltrating carcinoma, and the biomarker gene(s) is/are RARB and/or RASSF1A.

In some preferred embodiments, the breast cancer status is an infiltrating ductal carcinoma, and the biomarker gene(s) is/are APC, CCND2 and/or RASSF1A.

In some preferred embodiments, the breast cancer status is an infiltrating lobular carcinoma, and the biomarker gene(s) is/are CCND2 and/or RASSF1A.

In some preferred embodiments, the breast cancer status is an infiltrating carcinoma other type, and the biomarker gene(s) is/are CCND2 and/or RARB.

In some embodiments, step 2) comprises detecting the methylation level(s) of a target region within the biomarker gene(s), and wherein the target region is a nucleotide sequence of at least 15 bases in the biomarker gene(s), or a complementary sequence thereof.

In some embodiments, in step 2),
the detection of the methylation level of the APC gene comprises the use of a primer pair having the sequences as set forth in SEQ ID NOs: 12 and 13 or a primer pair having the sequences as set forth in SEQ ID NOs: 16 and 17 to carry out a PCR amplification reaction, with the APC gene or a fragment thereof which is bisulfite-treated in the biological sample as a template;
the detection of the methylation level of the BRCA1 gene comprises the use of a primer pair having the sequences as set forth in SEQ ID NOs: 20 and 21, a primer pair having the sequences as set forth in SEQ ID NOs: 24 and 25 or a primer pair having the sequences as set forth in SEQ ID NOs: 28 and 29 to carry out a PCR amplification reaction, with the BRCA1 gene or a fragment thereof which is bisulfite-treated in the biological sample as a template;
the detection of the methylation level of the CCND2 gene comprises the use of a primer pair having the sequences as set forth in SEQ ID NOs: 32 and 33, a primer pair having the sequences as set forth in SEQ ID NOs: 36 and 37 or a primer pair having the sequences as set forth in SEQ ID NOs: 40 and 41 to carry out a PCR amplification reaction, with the CCND2 gene or a fragment thereof which is bisulfite-treated in the biological sample as a template;
the detection of the methylation level of the CST6 gene comprises the use of a primer pair having the sequences as set forth in SEQ ID NOs: 44 and 45 or a primer pair having the sequences as set forth in SEQ ID NOs: 48 and 49 to carry out a PCR amplification reaction, with the CST6 gene or a fragment thereof which is bisulfite-treated in the biological sample as a template;
the detection of the methylation level of the GP5 gene comprises the use of a primer pair having the sequences as set forth in SEQ ID NOs: 52 and 53, a primer pair having the sequences as set forth in SEQ ID NOs: 56 and 57 or a primer pair having the sequences as set forth in SEQ ID NOs: 60 and 61 to carry out a PCR amplification reaction, with the GP5 gene or a fragment thereof which is bisulfite-treated in the biological sample as a template;
the detection of the methylation level of the GSTP1 gene comprises the use of a primer pair having the sequences as set forth in SEQ ID NOs: 64 and 65 or a primer pair having the sequences as set forth in SEQ ID NOs: 68 and 69 to carry out a PCR amplification reaction, with the GSTP1 gene or a fragment thereof which is bisulfite-treated in the biological sample as a template;
the detection of the methylation level of the PITX2 gene comprises the use of a primer pair having the sequences as set forth in SEQ ID NOs: 72 and 73, a primer pair having the sequences as set forth in SEQ ID NOs: 76 and 77, a primer pair having the sequences as set forth in SEQ ID NOs: 80 and 81 or a primer pair having the sequences as set forth in SEQ ID NOs: 84 and 85 to carry out a PCR amplification reaction, with the PITX2 gene or a fragment thereof which is bisulfite-treated in the biological sample as a template;
the detection of the methylation level of the RARB gene comprises the use of a primer pair having the sequences as set forth in SEQ ID NOs: 88 and 89, a primer pair having the sequences as set forth in SEQ ID NOs: 92 and 93 or a primer pair having the sequences as set forth in SEQ ID NOs: 96 and 97 to carry out a PCR amplification reaction, with the RARB gene or a fragment thereof which is bisulfite-treated in the biological sample as a template;
the detection of the methylation level of the RASSF1A gene comprises the use of a primer pair having the sequences as set forth in SEQ ID NOs: 100 and 101, a primer pair having the sequences as set forth in SEQ ID NOs: 104 and 105 or a primer pair having the sequences as set forth in SEQ ID NOs: 108 and 109 to carry out a PCR amplification reaction, with the RASSF1A gene or a fragment thereof which is bisulfite-treated in the biological sample as a template; and
the detection of the methylation level of the SOX17 gene comprises the use of a primer pair having the sequences as set forth in SEQ ID NOs: 112 and 113, a primer pair having the sequences as set forth in SEQ ID NOs: 116 and 117 or a primer pair having the sequences as set forth in SEQ ID NOs: 120 and 121 to carry out a PCR amplification reaction, with the SOX17 gene or a fragment thereof which is bisulfite-treated in the biological sample as a template.

In some preferred embodiments, in step 2),
the detection of the methylation level of the APC gene comprises the use of a primer pair having the sequences as set forth in SEQ ID NOs: 12 and 13 and a blocking primer having the sequence as set forth in SEQ ID NO: 14, or a primer pair having the sequences as set forth in SEQ ID NOs: 16 and 17 and a blocking primer having the sequence as set forth in SEQ ID NO: 18 to carry out a PCR amplification reaction, with the bisulfite-treated APC gene or a fragment thereof in the biological sample as a template;
the detection of the methylation level of the BRCA1 gene comprises the use of a primer pair having the sequences as set forth in SEQ ID NOs: 20 and 21 and a blocking primer having the sequence as set forth in SEQ ID NO: 22, a primer pair having the sequences as set forth in SEQ ID NOs: 24 and 25 and a blocking primer having the sequence as set forth in SEQ ID NO: 26 or a primer pair having the sequences as set forth in SEQ ID NOs: 28 and 29 and a blocking primer having the sequence as set forth in SEQ ID NO: 30 to carry out a PCR amplification reaction, with the bisulfite-treated BRCA1 gene or a fragment thereof in the biological sample as a template;
the detection of the methylation level of the CCND2 gene comprises the use of a primer pair having the sequences as set forth in SEQ ID NOs: 32 and 33 and a blocking primer having the sequence as set forth in SEQ ID NO: 34, a primer pair having the sequences as set forth in SEQ ID NOs: 36 and 37 and a blocking primer having the sequence as set forth in SEQ ID NO: 38 or a primer pair having the sequences as set forth in SEQ ID NOs: 40 and 41 and a blocking primer having the sequence as set forth in SEQ ID NO: 42 to carry out a PCR amplification reaction, with the bisulfite-treated CCND2 gene or a fragment thereof in the biological sample as a template;
the detection of the methylation level of the CST6 gene comprises the use of a primer pair having the sequences as set forth in SEQ ID NOs: 44 and 45 and a blocking primer having the sequence as set forth in SEQ ID NO: 46, or a primer pair having the sequences as set forth in SEQ ID NOs: 48 and 49 and a blocking primer having the sequence as set forth in SEQ ID NO: 50 to carry out a PCR amplification reaction, with the bisulfite-treated CST6 gene or a fragment thereof in the biological sample as a template;
the detection of the methylation level of the GP5 gene comprises the use of a primer pair having the sequences as set forth in SEQ ID NOs: 52 and 53 and a blocking primer having the sequence as set forth in SEQ ID NO: 54, a primer pair having the sequences as set forth in SEQ ID NOs: 56 and 57 and a blocking primer having the sequence as set forth in SEQ ID NO: 58 or a primer pair having the sequences as set forth in SEQ ID NOs: 60 and 61 and a blocking primer having the sequence as set forth in SEQ ID NO: 62 to carry out a PCR amplification reaction, with the bisulfite-treated GP5 gene or a fragment thereof in the biological sample as a template;
the detection of the methylation level of the GSTP1 gene comprises the use of a primer pair having the sequences as set forth in SEQ ID NOs: 64 and 65 and a blocking primer having the sequence as set forth in SEQ ID NO: 66, or a primer pair having the sequences as set forth in SEQ ID NOs: 68 and 69 and a blocking primer having the sequence as set forth in SEQ ID NO: 70 to carry out a PCR amplification reaction, with the bisulfite-treated GSTP1 gene or a fragment thereof in the biological sample as a template;
the detection of the methylation level of the PITX2 gene comprises the use of a primer pair having the sequences as set forth in SEQ ID NOs: 72 and 73 and a blocking primer having the sequence as set forth in SEQ ID NO: 74, a primer pair having the sequences as set forth in SEQ ID NOs: 76 and 77 and a blocking primer having the sequence as set forth in SEQ ID NO: 78, a primer pair having the sequences as set forth in SEQ ID NOs: 80 and 81 and a blocking primer having the sequence as set forth in SEQ ID NO: 82 or a primer pair having the sequences as set forth in SEQ ID NOs: 84 and 85 and a blocking primer having the sequence as set forth in SEQ ID NO: 86 to carry out a PCR amplification reaction, with the bisulfite-treated PITX2 gene or a fragment thereof in the biological sample as a template;
the detection of the methylation level of the RARB gene comprises the use of a primer pair having the sequences as set forth in SEQ ID NOs: 88 and 89 and a blocking primer having the sequence as set forth in SEQ ID NO: 90, a primer pair having the sequences as set forth in SEQ ID NOs: 92 and 93 and a blocking primer having the sequence as set forth in SEQ ID NO: 94, or a primer pair having the sequences as set forth in SEQ ID NOs: 96 and 97 and a blocking primer having the sequence as set forth in SEQ ID NO: 98 to carry out a PCR amplification reaction, with the bisulfite-treated RARB gene or a fragment thereof in the biological sample as a template;
the detection of the methylation level of the RASSF1A gene comprises the use of a primer pair having the sequences as set forth in SEQ ID NOs: 100 and 101 and a blocking primer having the sequence as set forth in SEQ ID NO: 102, or a primer pair having the sequences as set forth in SEQ ID NOs: 104 and 105 and a blocking primer having the sequence as set forth in SEQ ID NO: 106 to carry out a PCR amplification reaction, with the bisulfite-treated RASSF1A gene or a fragment thereof in the biological sample as a template; and
the detection of the methylation level of the SOX17 gene comprises the use of a primer pair having the sequences as set forth in SEQ ID NOs: 112 and 113 and a blocking primer having the sequence as set forth in SEQ ID NO: 114, a primer pair having the sequences as set forth in SEQ ID NOs: 116 and 117 and a blocking primer having the sequence as set forth in SEQ ID NO: 118 or a primer pair having the sequences as set forth in SEQ ID NOs: 120 and 121 and a blocking primer having the sequence as set forth in SEQ ID NO: 122 to carry out a PCR amplification reaction, with the bisulfite-treated SOX17 gene or a fragment thereof in the biological sample as a template,
wherein the blocking primers have a 3' end modification which prevents the extension and amplification of a DNA polymerase.

In more preferred embodiments, in step 2),
the detection of the methylation level of the APC gene comprises the use of a primer pair having the sequences as set forth in SEQ ID NOs: 12 and 13, a blocking primer having the sequence as set forth in SEQ ID NO: 14 and a probe having the sequence as set forth in SEQ ID NO: 15; or a primer pair having the sequences as set forth in SEQ ID NOs: 16 and 17, a blocking primer having the sequence as set forth in SEQ ID NO: 18 and a probe having the sequence as set forth in SEQ ID NO: 19 to carry out a PCR amplification reaction, with the bisulfite-treated APC gene or a fragment thereof in the biological sample as a template;
the detection of the methylation level of the BRCA1 gene comprises the use of a primer pair having the sequences as set forth in SEQ ID NOs: 20 and 21, a blocking primer having the sequence as set forth in SEQ ID NO: 22 and a probe having the sequence as set forth in SEQ ID NO: 23; a primer pair having the sequences as set forth in SEQ ID NOs: 24 and 25, a blocking primer having the sequence as set forth in SEQ ID NO: 26 and a probe having the sequence as set forth in SEQ ID NO: 27; or a primer pair having the sequences as set forth in SEQ ID NOs: 28 and 29, a blocking primer having the sequence as set forth in SEQ ID NO: 30 and a probe having the sequence as set forth in SEQ ID NO: 31 to carry out a PCR amplification reaction, with the bisulfite-treated BRCA1 gene or a fragment thereof in the biological sample as a template;
the detection of the methylation level of the CCND2 gene comprises the use of a primer pair having the sequences as set forth in SEQ ID NOs: 32 and 33, a blocking primer having the sequence as set forth in SEQ ID NO: 34 and a probe having the sequence as set forth in SEQ ID NO: 35; a primer pair having the sequences as set forth in SEQ ID NOs: 36 and 37, a blocking primer having the sequence as set forth in SEQ ID NO: 38 and a probe having the sequence as set forth in SEQ ID NO: 39; or a primer pair having the sequences as set forth in SEQ ID NOs: 40 and 41, a blocking primer having the sequence as set forth in SEQ ID NO: 42 and a probe having the sequence as set forth in SEQ ID NO: 43 to carry out a PCR amplification reaction, with the bisulfite-treated CCND2 gene or a fragment thereof in the biological sample as a template;
the detection of the methylation level of the CST6 gene comprises the use of a primer pair having the sequences as set forth in SEQ ID NOs: 44 and 45, a blocking primer having the sequence as set forth in SEQ ID NO: 46 and a probe having the sequence as set forth in SEQ ID NO: 47; or a primer pair having the sequences as set forth in SEQ ID NOs: 48 and 49, a blocking primer having the sequence as set forth in SEQ ID NO: 50 and a probe having the sequence as set forth in SEQ ID NO: 51 to carry out a PCR amplification reaction, with the bisulfite-treated CST6 gene or a fragment thereof in the biological sample as a template;
the detection of the methylation level of the GP5 gene comprises the use of a primer pair having the sequences as set forth in SEQ ID NOs: 52 and 53, a blocking primer having the sequence as set forth in SEQ ID NO: 54 and a probe having the sequence as set forth in SEQ ID NO: 55; a primer pair having the sequences as set forth in SEQ ID NOs: 56 and 57, a blocking primer having the sequence as set forth in SEQ ID NO: 58 and a probe having the sequence as set forth in SEQ ID NO: 59; or a primer pair having the sequences as set forth in SEQ ID NOs: 60 and 61, a blocking primer having the sequence as set forth in SEQ ID NO: 62 and a probe having the sequence as set forth in SEQ ID NO: 63 to carry out a PCR amplification reaction, with the bisulfite-treated GP5 gene or a fragment thereof in the biological sample as a template;
the detection of the methylation level of the GSTP1 gene comprises the use of a primer pair having the sequences as set forth in SEQ ID NOs: 64 and 65, a blocking primer having the sequence as set forth in SEQ ID NO: 66 and a probe having the sequence as set forth in SEQ ID NO: 67; or a primer pair having the sequences as set forth in SEQ ID NOs: 68 and 69, a blocking primer having the sequence as set forth in SEQ ID NO: 70 and a probe having the sequence as set forth in SEQ ID NO: 71 to carry out a PCR amplification reaction, with the bisulfite-treated GSTP1 gene or a fragment thereof in the biological sample as a template;
the detection of the methylation level of the PITX2 gene comprises the use of a primer pair having the sequences as set forth in SEQ ID NOs: 72 and 73, a blocking primer having the sequence as set forth in SEQ ID NO: 74 and a probe having the sequence as set forth in SEQ ID NO: 75; a primer pair having the sequences as set forth in SEQ ID NOs: 76 and 77, a blocking primer having the sequence as set forth in SEQ ID NO: 78 and a probe having the sequence as set forth in SEQ ID NO: 79; a primer pair having the sequences as set forth in SEQ ID NOs: 80 and 81, a blocking primer having the sequence as set forth in SEQ ID NO: 82 and a probe having the sequence as set forth in SEQ ID NO: 83; or a primer pair having the sequences as set forth in SEQ ID NOs: 84 and 85, a blocking primer having the sequence as set forth in SEQ ID NO: 86 and a probe having the sequence as set forth in SEQ ID NO: 87to carry out a PCR amplification reaction, with the bisulfite-treated PITX2 gene or a fragment thereof in the biological sample as a template;
the detection of the methylation level of the RARB gene comprises the use of a primer pair having the sequences as set forth in SEQ ID NOs: 88 and 89, a blocking primer having the sequence as set forth in SEQ ID NO: 90 and a probe having the sequence as set forth in SEQ ID NO: 91; a primer pair having the sequences as set forth in SEQ ID NOs: 92 and 93, a blocking primer having the sequence as set forth in SEQ ID NO: 94 and a probe having the sequence as set forth in SEQ ID NO: 95; or a primer pair having the sequences as set forth in SEQ ID NOs: 96 and 97, a blocking primer having the sequence as set forth in SEQ ID NO: 98 and a probe having the sequence as set forth in SEQ ID NO: 99 to carry out a PCR amplification reaction, with the bisulfite-treated RARB gene or a fragment thereof in the biological sample as a template;
the detection of the methylation level of the RASSF1A gene comprises the use of a primer pair having the sequences as set forth in SEQ ID NOs: 100 and 101, a blocking primer having the sequence as set forth in SEQ ID NO: 102 and a probe having the sequence as set forth in SEQ ID NO: 103; a primer pair having the sequences as set forth in SEQ ID NOs: 104 and 105, a blocking primer having the sequence as set forth in SEQ ID NO: 106 and a probe having the sequence as set forth in SEQ ID NO: 107; or a primer pair having the sequences as set forth in SEQ ID NOs: 108 and 109, a blocking primer having the sequence as set forth in SEQ ID NO: 110 and a probe having the sequence as set forth in SEQ ID NO: 111 to carry out a PCR amplification reaction, with the bisulfite-treated RASSF1A gene or a fragment thereof in the biological sample as a template; and
the detection of the methylation level of the SOX17 gene comprises the use of a primer pair having the sequences as set forth in SEQ ID NOs: 112 and 113, a blocking primer having the sequence as set forth in SEQ ID NO: 114 and a probe having the sequence as set forth in SEQ ID NO: 115; a primer pair having the sequences as set forth in SEQ ID NOs: 116 and 117, a blocking primer having the sequence as set forth in SEQ ID NO: 118 and a probe having the sequence as set forth in SEQ ID NO: 119; or a primer pair having the sequences as set forth in SEQ ID NOs: 120 and 121 a blocking primer having the sequence as set forth in SEQ ID NO: 122 and a probe having the sequence as set forth in SEQ ID NO: 123 to carry out a PCR amplification reaction, with the bisulfite-treated SOX17 gene or a fragment thereof in the biological sample as a template,
wherein the probes have a fluorescent group at one end and a fluorescence quenching group at the other end.

In some embodiments, step 2) further comprises the use of a primer pair having the sequences as set forth in SEQ ID NOs: 124 and 125 and a probe having the sequence as set forth in SEQ ID NO: 126 to carry out a PCR amplification reaction, with a bisulfite-treated ACTB gene or a fragment thereof used as an internal reference gene in the biological sample as a template.

In some embodiments, step 3) comprises determining the breast cancer status in the subject according to the methylation levels of the biomarker genes based on a logistic regression.

In another aspect, a kit for identifying a breast cancer status in a subject is provided herein, which comprises a primer pair for detecting methylation level(s) of a biomarker gene in a biological sample from the subject, wherein the primer pair is used to carry out a PCR amplification reaction with the biomarker gene or a fragment thereof which is bisulfite-treated as a template; and the biomarker gene(s) is/are selected from one or more of the following genes: APC, BRCA1, CCND2, CST6, GP5, GSTP1, PITX2, RARB, RASSF1A and SOX17.

In some preferred enbodiments, the biomarker genes are selected from 2 or more of APC, BRCA1, CCND2, CST6, GP5, GSTP1, PITX2, RARB, RASSF1A and SOX17.

In more preferred enbodiments, the biomarker genes are selected from 5 or more of APC, BRCA1, CCND2, CST6, GP5, GSTP1, PITX2, RARB, RASSF1A and SOX17.

In some particular enbodiments, the biomarker genes are BRCA1, CCND2, PITX2, RARB and RASSF1A.

In some enbodiments, the breast cancer status is breast cancer stage I or stage II, and the biomarker gene(s) is/are APC and/or RASSF1A. In some enbodiments, the breast cancer status is a noninfiltrating carcinoma, and the biomarker gene(s) is/are RARB and/or RASSF1A. In some enbodiments, the breast cancer status is an infiltrating ductal carcinoma, and the biomarker gene(s) is/are APC, CCND2 and/or RASSF1A. In some enbodiments, the breast cancer status is an infiltrating lobular carcinoma, and the biomarker gene(s) is/are CCND2 and/or RASSF1A. In some enbodiments, the breast cancer status is an infiltrating carcinoma of other type, and the biomarker gene(s) is/are CCND2 and/or RARB.

In some enbodiments, in the kit, the primer pair used for the the detection of the methylation level of APC has the sequences as set forth in SEQ ID NOs: 12 and 13 or the sequences as set forth in SEQ ID NOs: 16 and 17;
the primer pair used for the the detection of the methylation level of BRCA1 has the sequences as set forth in SEQ ID NOs: 20 and 21, the sequences as set forth in SEQ ID NOs: 24 and 25 or the sequences as set forth in SEQ ID NOs: 28 and 29;
the primer pair used for the the detection of the methylation level of CCND2 has the sequences as set forth in SEQ ID NOs: 32 and 33, the sequences as set forth in SEQ ID NOs: 36 and 37 or the sequences as set forth in SEQ ID NOs: 40 and 41;
the primer pair used for the the detection of the methylation level of CST6 has the sequences as set forth in SEQ ID NOs: 44 and 45 or the sequences as set forth in SEQ ID NOs: 48 and 49;
the primer pair used for the the detection of the methylation level of GP5 has the sequences as set forth in SEQ ID NOs: 52 and 53, the sequences as set forth in SEQ ID NOs: 56 and 57 or has the sequences as set forth in SEQ ID NOs: 60 and 61;
the primer pair used for the the detection of the methylation level of GSTP1 has the sequences as set forth in SEQ ID NOs: 64 and 65 or the sequences as set forth in SEQ ID NOs: 68 and 69;
the primer pair used for the the detection of the methylation level of PITX2 has the sequences as set forth in SEQ ID NOs: 72 and 73, the sequences as set forth in SEQ ID NOs: 76 and 77, the sequences as set forth in SEQ ID NOs: 80 and 81 or the sequences as set forth in SEQ ID NOs: 84 and 85;
the primer pair used for the the detection of the methylation level of RARB has the sequences as set forth in SEQ ID NOs: 88 and 89, the sequences as set forth in SEQ ID NOs: 92 and 93 or the sequences as set forth in SEQ ID NOs: 96 and 97;
the primer pair used for the the detection of the methylation level of RASSF1A has the sequences as set forth in SEQ ID NOs: 100 and 101, the sequences as set forth in SEQ ID NOs: 104 and 105 or the sequences as set forth in SEQ ID NOs: 108 and 109; and
the primer pair used for the the detection of the methylation level of SOX17 has the sequences as set forth in SEQ ID NOs: 112 and 113, the sequences as set forth in SEQ ID NOs: 116 and 117 or the sequences as set forth in SEQ ID NOs: 120 and 121.

In some enbodiments, the kit can further comprise a blocking primer, wherein the blocking primer used in combination with the primer pair having the sequences as set forth in SEQ ID NOs: 12 and 13 has the sequence as set forth in SEQ ID NO: 14;
the blocking primer used in combination with the primer pair having the sequences as set forth in SEQ ID NOs: 16 and 17 has the sequence as set forth in SEQ ID NO: 18;
the blocking primer used in combination with the primer pair having the sequences as set forth in SEQ ID NOs: 20 and 21 has the sequence as set forth in SEQ ID NO: 22;
the blocking primer used in combination with the primer pair having the sequences as set forth in SEQ ID NOs: 24 and 25 has the sequence as set forth in SEQ ID NO: 26;
the blocking primer used in combination with the primer pair having the sequences as set forth in SEQ ID NOs: 28 and 29 has the sequence as set forth in SEQ ID NO: 30;
the blocking primer used in combination with the primer pair having the sequences as set forth in SEQ ID NOs: 32 and 33 has the sequence as set forth in SEQ ID NO: 34;
the blocking primer used in combination with the primer pair having the sequences as set forth in SEQ ID NOs: 36 and 37 has the sequence as set forth in SEQ ID NO: 38;
the blocking primer used in combination with the primer pair having the sequences as set forth in SEQ ID NOs: 40 and 41 has the sequence as set forth in SEQ ID NO: 42;
the blocking primer used in combination with the primer pair having the sequences as set forth in SEQ ID NOs: 44 and 45 has the sequence as set forth in SEQ ID NO: 46;
the blocking primer used in combination with the primer pair having the sequences as set forth in SEQ ID NOs: 48 and 49 has the sequence as set forth in SEQ ID NO: 50;
the blocking primer used in combination with the primer pair having the sequences as set forth in SEQ ID NOs: 52 and 53 has the sequence as set forth in SEQ ID NO: 54;
the blocking primer used in combination with the primer pair having the sequences as set forth in SEQ ID NOs: 56 and 57 has the sequence as set forth in SEQ ID NO: 58;
the blocking primer used in combination with the primer pair having the sequences as set forth in SEQ ID NOs: 60 and 61 has the sequence as set forth in SEQ ID NO: 62;
the blocking primer used in combination with the primer pair having the sequences as set forth in SEQ ID NOs: 64 and 65 has the sequence as set forth in SEQ ID NO: 66;
the blocking primer used in combination with the primer pair having the sequences as set forth in SEQ ID NOs: 68 and 69 has the sequence as set forth in SEQ ID NO: 70;
the blocking primer used in combination with the primer pair having the sequences as set forth in SEQ ID NOs: 72 and 73 has the sequence as set forth in SEQ ID NO: 74;
the blocking primer used in combination with the primer pair having the sequences as set forth in SEQ ID NOs: 76 and 77 has the sequence as set forth in SEQ ID NO: 78;
the blocking primer used in combination with the primer pair having the sequences as set forth in SEQ ID NOs: 80 and 81 has the sequence as set forth in SEQ ID NO: 82;
the blocking primer used in combination with the primer pair having the sequences as set forth in SEQ ID NOs: 84 and 85 has the sequence as set forth in SEQ ID NO: 86;
the blocking primer used in combination with the primer pair having the sequences as set forth in SEQ ID NOs: 88 and 89 has the sequence as set forth in SEQ ID NO: 90;
the blocking primer used in combination with the primer pair having the sequences as set forth in SEQ ID NOs: 92 and 93 has the sequence as set forth in SEQ ID NO: 94;
the blocking primer used in combination with the primer pair having the sequences as set forth in SEQ ID NOs: 96 and 97 has the sequence as set forth in SEQ ID NO: 98;
the blocking primer used in combination with the primer pair having the sequences as set forth in SEQ ID NOs: 100 and 101 has the sequence as set forth in SEQ ID NO: 102;
the blocking primer used in combination with the primer pair having the sequences as set forth in SEQ ID NOs: 104 and 105 has the sequence as set forth in SEQ ID NO: 106;
the blocking primer used in combination with the primer pair having the sequences as set forth in SEQ ID NOs: 108 and 109 has the sequence as set forth in SEQ ID NO: 110;
the blocking primer used in combination with the primer pair having the sequences as set forth in SEQ ID NOs: 112 and 113 has the sequence as set forth in SEQ ID NO: 114;
the blocking primer used in combination with the primer pair having the sequences as set forth in SEQ ID NOs: 116 and 117 has the sequence as set forth in SEQ ID NO: 118; and
the blocking primer used in combination with the primer pair having the sequences as set forth in SEQ ID NOs: 120 and 121 has the sequence as set forth in SEQ ID NO: 122;
wherein the blocking primers have a 3' end modification which prevents the extension and amplification of a DNA polymerase.

In some enbodiments, the kit can further comprise a probe, wherein the probe used in combination with the primer pair having the sequences as set forth in SEQ ID NOs: 12 and 13 has the sequence as set forth in SEQ ID NO: 15;
the probe used in combination with the primer pair having the sequences as set forth in SEQ ID NOs: 16 and 17 has the sequence as set forth in SEQ ID NO: 19;
the probe used in combination with the primer pair having the sequences as set forth in SEQ ID NOs: 20 and 21 has the sequence as set forth in SEQ ID NO: 23;
the probe used in combination with the primer pair having the sequences as set forth in SEQ ID NOs: 24 and 25 has the sequence as set forth in SEQ ID NO: 27;
the probe used in combination with the primer pair having the sequences as set forth in SEQ ID NOs: 28 and 29 has the sequence as set forth in SEQ ID NO: 31;
the probe used in combination with the primer pair having the sequences as set forth in SEQ ID NOs: 32 and 33 has the sequence as set forth in SEQ ID NO: 35;
the probe used in combination with the primer pair having the sequences as set forth in SEQ ID NOs: 36 and 37 has the sequence as set forth in SEQ ID NO: 39;
the probe used in combination with the primer pair having the sequences as set forth in SEQ ID NOs: 40 and 41 has the sequence as set forth in SEQ ID NO: 43;
the probe used in combination with the primer pair having the sequences as set forth in SEQ ID NOs: 44 and 45 has the sequence as set forth in SEQ ID NO: 47;
the probe used in combination with the primer pair having the sequences as set forth in SEQ ID NOs: 48 and 49 has the sequence as set forth in SEQ ID NO: 51;
the probe used in combination with the primer pair having the sequences as set forth in SEQ ID NOs: 52 and 53 has the sequence as set forth in SEQ ID NO: 55;
the probe used in combination with the primer pair having the sequences as set forth in SEQ ID NOs: 56 and 57 has the sequence as set forth in SEQ ID NO: 59;
the probe used in combination with the primer pair having the sequences as set forth in SEQ ID NOs: 60 and 61 has the sequence as set forth in SEQ ID NO: 63;
the probe used in combination with the primer pair having the sequences as set forth in SEQ ID NOs: 64 and 65 has the sequence as set forth in SEQ ID NO: 67;
the probe used in combination with the primer pair having the sequences as set forth in SEQ ID NOs: 68 and 69 has the sequence as set forth in SEQ ID NO: 71;
the probe used in combination with the primer pair having the sequences as set forth in SEQ ID NOs: 72 and 73 has the sequence as set forth in SEQ ID NO: 75;
the probe used in combination with the primer pair having the sequences as set forth in SEQ ID NOs: 76 and 77 has the sequence as set forth in SEQ ID NO: 79;
the probe used in combination with the primer pair having the sequences as set forth in SEQ ID NOs: 80 and 81 has the sequence as set forth in SEQ ID NO: 83;
the probe used in combination with the primer pair having the sequences as set forth in SEQ ID NOs: 84 and 85 has the sequence as set forth in SEQ ID NO: 87;
the probe used in combination with the primer pair having the sequences as set forth in SEQ ID NOs: 88 and 89 has the sequence as set forth in SEQ ID NO: 91;
the probe used in combination with the primer pair having the sequences as set forth in SEQ ID NOs: 92 and 93 has the sequence as set forth in SEQ ID NO: 95;
the probe used in combination with the primer pair having the sequences as set forth in SEQ ID NOs: 96 and 97 has the sequence as set forth in SEQ ID NO: 99;
the probe used in combination with the primer pair having the sequences as set forth in SEQ ID NOs: 100 and 101 has the sequence as set forth in SEQ ID NO: 103;
the probe used in combination with the primer pair having the sequences as set forth in SEQ ID NOs: 104 and 105 has the sequence as set forth in SEQ ID NO: 107;
the probe used in combination with the primer pair having the sequences as set forth in SEQ ID NOs: 108 and 109 has the sequence as set forth in SEQ ID NO: 111;
the probe used in combination with the primer pair having the sequences as set forth in SEQ ID NOs: 112 and 113 has the sequence as set forth in SEQ ID NO: 115;
the probe used in combination with the primer pair having the sequences as set forth in SEQ ID NOs: 116 and 117 has the sequence as set forth in SEQ ID NO: 119; and
the probe used in combination with the primer pair having the sequences as set forth in SEQ ID NOs: 120 and 121 has the sequence as set forth in SEQ ID NO: 123,
wherein the probes have a fluorescent group at one end and a fluorescence quenching group at the other end.

In more preferred enbodiments, the kit comprises the primer pair and the corresponding blocking primer and probe.

In some enbodiments, the kit further comprises a primer pair having the sequences as set forth in SEQ ID NOs: 124 and 125 and a probe having the sequence as set forth in SEQ ID NO: 126, for carrying out a PCR amplification reaction with a bisulfite-treated ACTB gene or a fragment thereof used as an internal reference gene in the biological sample as a template.

In preferred enbodiments, the kit further comprises a DNA extraction reagent and a bisulfite reagent. Preferably, the bisulfite reagent comprises sodium bisulfite.

In preferred embodiments, the kit further comprises an instruction that discribes how to use the kit and process detection results with a logistic regression.

The breast cancer status includes the breast cancer susceptibility and the presence, progression, subtype, and/or stage of the breast cancer.

The biological sample is selected from blood, serum, plasma, breast duct fluid, lymph, cerebrospinal fluid, urine, and tissue biopsy from the subject.

The method and kit provided by the present application provide a fast, reliable, and accurate new way for the prediction, diagnosis, and evaluation of a breast cancer.

### BRIEF DESCRIPTION OF THE DRAWINGS

Figure 1 shows the receiver operating characteristic (ROC) curves of the methylation levels of 10 biomarker genes.
Figure 2 shows the methylation level distribution of 10 biomarker genes in different breast cancer stages (indicated by Ct values). Figure 2A shows the methylation level distribution of APC, BRCA1, CCND2, CST6, GP5 and GSTP1, and Figure 2B shows the methylation level distribution of PITX2, RARB, RASSF1A and SOX17.
Figure 3 shows the methylation level distribution of 10 biomarker genes in different breast cancer subtypes (indicated by Ct values). Figure 3A shows the methylation level distribution of APC, BRCA1, CCND2, CST6, GP5 and GSTP1, and Figure 2B shows the methylation level distribution of PITX2, RARB, RASSF1A and SOX17.
Figure 4 shows the receiver operating characteristic (ROC) curve of a logistic regression model constructed with 10 marker genes;
Figure 5 shows the receiver operating characteristic (ROC) curve of a logistic regression model constructed with the 5 most characteristic marker genes.

### DETAILED DESCRIPTION

Unless otherwise stated, the technical terms used in this application have the meanings generally understood by those skilled in the art to which the present invention belongs.

The present application in one aspect relates to a method for identifying a breast cancer status in a subject, which comprises the following steps: 1) collecting a biological sample from the subject; 2) detecting the methylation level(s) of a biomarker gene in the biological sample, wherein the biomarker gene(s) is/are selected from one or more of the following genes: APC (adenomatosis polyposis coli), BRCA1 (Breast Cancer 1 protein), CCND2 (cyclin D2), CST6 (cystatin E/M), GP5 (glycoprotein V platelet), GSTP1 (glutathione S-transferase pi 1), PITX2 (paired like homeodomain 2), RARB (retinoic acid receptor beta), RASSF1A (Ras association domain family member 1) and SOX17 (SRY-box 17); and 3) comparing the methylation levels detected in step 2) with the normal methylation levels of the corresponding biomarker gene(s) in a population to determine the breast cancer status in the subject.

The term "subject" as used herein refers to an individual (preferably a human) suffering from or suspected of having a certain disease, or, when predicting the susceptibility, "subject" may also include healthy individuals. The term is generally used interchangeably with "patient", "test subject", "treatment subject", and the like.

The term "population" as used herein generally refers to healthy people. When referring to a specific disease (such as a breast cancer), a "population" may include individuals who do not suffer from the specific disease but may suffer from other diseases. In addition, it is also possible to select only some individuals as the "population" based on characteristics such as, age, gender, health status, in menopause or not, etc. A "normal methylation level in a population" can be obtained by detecting enough individuals or can be found in an existing clinical literature. In some cases, this normal level refers to no methylation.

The term "breast cancer status" used herein includes a breast cancer susceptibility and the presence, progression, subtype, and/or stage of a breast cancer. In some embodiments, the subject's susceptibility to a breast cancer can be predicted based on the methylation levels of the biomarker gene(s) in the subject. In other embodiments, the subject may be identified for the presence of a breast cancer based on the methylation levels of the biomarker gene(s) in the subject; and if a breast cancer is present, the subtype and/or the stage of the breast cancer may be identified. Breast cancer subtypes may include noninfiltrating carcinomas, infiltrating ductal carcinomas, infiltrating lobular carcinomas, and other infiltrating carcinomas. The breast cancer stages may include stage I (IA, IB, or IC), stage II, stage III, and stage IV. In some embodiments, the breast cancer is a stage I breast cancer. In some embodiments, the breast cancer is a stage II breast cancer. In some embodiments, the breast cancer is a stage III breast cancer. In other embodiments, the breast cancer is a stage IV breast cancer.

In the method of the present invention, treatment of the subject, for example, including performing more tests on the subject, performing a surgery, giving medications, and taking no further actions, may also be arranged based on the stage of the breast cancer. In other embodiments, the method of the present invention further comprises measuring the methylation levels of one or more biomarker genes of APC, BRCA1, CCND2, CST6, GP5, GSTP1, PITX2, RARB, RASSF1A and SOX17 genes or fragments thereof in the subject after the subject is treated, and correlating the measurement results with the breast cancer status to identify whether the treatment results in a change in the breast cancer status in the subject. In some embodiments, the correlation is performed by a classification algorithm of a software.

The detection of the methylation levels in step 2) comprises extracting DNA from a biological sample, treating it with a bisulfite, and then carrying out a PCR amplification reaction by using a methylation-specific primer pair. The bisulfite treatment causes unmethylated cytosine residues in a double-stranded DNA molecule to deaminate to be uracils; while methylated cytosine residues remain unchanged. As a result, in the subsequent PCR amplification reaction, methylated cytosine residue sites on a template are paired with guanine residues in a primer as cytosine residues, while unmethylated cytosine residue sites are paired with adenine residues in a primer as uracil residues. The inventors designed multiple primer pairs for each biomarker gene to detect the methylation level of a target region within each biomarker gene. The target regions are selected from the fragments of at least 15 consecutive bases in the sequences as set forth in SEQ ID NOs: 1-11, respectively; and the nucleic acid sequences of the primer pairs are respectively identical, complementary or hybridizable to the above target regions. The primer pairs provided herein make use of the methylation difference to detect the methylation levels of the target regions within the biomarker genes. When a target region of a biomarker gene is not methylated, the primer pair used cannot effectively pair with and bind to the target region (treated with bisulfite) which is used as a template in the PCR amplification reaction, and cannot (or rarely) generate amplification products; and when the target gene of the biomarker gene is methylated, the primer pair used is able to effectively pair with and bind to the target region (treated with bisulfite) which is used as a template in the PCR amplification reaction, and thus generate amplification products. The differences of these amplification reactions can be monitored in real time during the amplification reactions, or can be judged by detecting the amplification products. After many experiments, the inventors screened out multiple primer pairs for the biomarker genes (see below), which can be used alone or in combination to help identify the breast cancer status in the subject.

| |
|---|
| SEQ ID NO:1 DNA sequence of human APC |
| GAGATGTAATTTATTACTCTCCCTCCCACCTCCGGCATCTTGTGCTAATCCTTCTGCCCTGCGGACCTCCCCCGACTCT TTACTATGCGTGTCAACTGCCATCAACTTCCTTGCTTGCTGGGGACTGGGGCCGCGAGGGCATACCCCCGAGGGGT ACGGGGCTAGGGCTAGGCAGGCTGTGCGGTTGGGCGGGGCCCTGTGCCCCACTGCGGAGTGCGGGTCGGGAAG CGGAGAGAGAAGCAGCTGTGTAATCCGCTGGATGCGGACCAGGGCGCTCCCCATTCCCGTCGGGAGCCCGCCGAT TGGCTGGGTGTGGGCGCACGTGACCGACATGTGGCTGTATTGGTGCAGCCCGCCAGGGTGTCACTGGAGACAGAA TGGAGGTGCTGCCGGACTCGGAAATGGGGTAGGTGCTGGAGCCACCATGGCCAGGCTTGCTGCGGGGGGAGGGG GGAAGGTGGTTTTCCCTCGCACTGTCTTAAA |

| |
|---|
| SEQ ID NO:2 DNA sequence of human BRCA1 |
| CCTCTTCCGTCTCTTTCCTTTTACGTCATCCGGGGGCAGACTGGGTGGCCAATCCAGAGCCCCGAGAGACGCTTGG CTCTTTCTGTCCCTCCCATCCTCTGATTGTACCTTGATTTCGTATTCTGAGAGGCTGCTGCTTAGCGGTAGCCCCTTGG TTTCCGTGGCAACGGAAAAGCGCGGGAATTACAGATAAATTAAAACTGCGACTGCGCGGCGTGAGCTCGCTGAGAC TTCCTGGACGGGGGACAGGCTGTGGGGTTTCTCAGATAACTGGGCCCCTGCGCTCAGGAGGCCTTCACCCTCTGCT CTGGGTAAAGGTAGTAGAGTCCCGGGAAAGGACAGGGGGCCCAAGTGATGCTCTGGGGTACTGGCGTGGGAGA GTGGATTTCCGAAGCTGACAGATGGGTATTCTTTGACGGGGGGTAGGGGCGGAACCTGAGAGGCGTAAGGCGTTG TGAACCCTGGGGAGGGGGGCAGTTTGTAGGTCGCGAGGGAAGCGCTGAGGATCAGGAAGGGGGCACTGAGTGTC CGTGGGGGAATCCTCGTGATAGGAACTGGAATATGCCTTGAGGGGGACACTATGTCTTTAAAAACGTCGGCTGGTCA TGAGGTCAGGAGTTCCAGACCAGCCTGACCAACGTGGTGAAACTCCGTCTCTACTAAAAATACAAAAATTAGCCGGG |
| CGTGGTGCCGCTCCAGCTACTCAGGAGGCTGAGGCAGGAGAATCGCTAGAACCCGGGAGGCGGAGGTTGCAGTG AGCCGAGATCGCGCCATTGCACTCCAGCCTGGGCGACAGAGCGAGACTGTCTCAAAACAAAACAAAACAA |

| |
|---|
| SEQ ID NO:3 DNA sequence of human CCND2 |
| AAGAGGCTGGCTCCGGGCCTGAGTTAATCGCTTGCACCTCTAGTTTATTCGCTCCCTCCTCCGCCTTGCAGGGAA CCTAGTGTACGGCTCACCCAGCCCGCGCCCCACCCCGCCTTGCTGGCTCTCCGCGCCCCTGCCCGGGCCCCCTCTC TCGGTGAGGGAGGCACTCAGTCGGCCTCGGTGTGCCCAGAGAGCTCGAGCCACGCCATGCCCGCTGCACGTGCC AGCTTGGCCAGCACATCAGGGCGCTGGTCTCTCCCCTTCCTCCTGGAGTGAAATACACCAAAGGGCGCGGTGGGG GTGGGGGGTGACGGGAGGAAGGAGGTGAAGAAACGCCACCAGATCGTATCTCCTGTAAAGACAGCCTTGACTCAA GCATGCGTTAGAGCACGTGTCAGGGCCGACCGTGCTGGCGGCGACTTCACCGCAGTCGGCTCCCAGGGAGAAAGC CTGGCGAGTGAGGCGCGAAACCGGAGGGGTCGGCGAGGATGCGGGCGAAGGACCGAGCGTGGAGGCCTCATGC CTCCGGGGAAAGGAAGGGGTGGTGGTGTTTGCGCAGGGGGAGCGAGGGGGAGCCGGACCTAATCCCTCACTCGC CCCCTCCCCCTCCCGGGCCATTTCCTAGAAAGCTGCATCGGTGTGGCCACGCTCAGCGCAGACACCTCGGGCGGCT TGTCAGCAGATGCAGGGGCGAGGAAGCGGGTTTTTCCTGCGTGGCCGCTGGCCGCGGGGGAACCGCTGGGAGCC CTGCCCCCGGCCTGCGGCGGCCCTAGACGCTGCACCGCGTCGCCCCACGGCGCCCGAAGAGCCCCCAGAAACAC GATGGTTTCTGCTCGAGGATCACATTCTATCCCTCCAGAGAAGCACCCCCCTTCCTTCCTAATACCCACCTCTC |

| |
|---|
| SEQ ID NO:4 DNA sequence of human CST6 |
| GTAAGAGCCCACGAAGAGCTGCGGTTGGTAGTTCATTCTGGACAGCCCTCCCGTGAACCGTCCCTGTACTGGCACT TGTTGCTGGGGACTGTCGCTGTCCTCTCCCTCCCCGGCCAGGTGTGTCCTGGAGGGCAGGGAAGCGTCTTGGCA CGCGGGTGCGCGCCGCCCCCTCGGCCTCCTGGGCTCCCTGAACCTCGCAGGACCCCGGCAACTTCGAGCCCCGC CCCAGCTCCAGGCCGCGGGGGCGCATCGCGGGCGTCGGGCGGGGCGGCCCAGCGGGTAAAAGCTGCGCGGCCG CAAGCTCGGCACTCACGGCTCTGAGGGCTCCGACGGCACTGACGGCCATGGCGCGTTCGAACCTCCCGCTGGCGC TGGGCCTGGCCCTGGTCGCATTCTGCCTCCTGGCGCTGCCACGCGACGCCCGGGCCCGGCCGCAGGAGCGCATG GTCGGAGAACTCCGGGACCTGTCGCCCGACGACCCGCAGGTGCAGAAGGCGGCGCAGGCGGCCGTGGCCAGCTA CAACATGGGCAGCAACAGCATCTACTACTTCCGAGACACGCACATCATCAAGGCGCAGAGCCAGGTGCGGCGGGC GGGGTGCTGGGAGGGGACACCCGGC |

| |
|---|
| SEQ ID NO:5 DNA sequence of human GP5 |
| CCGCGGGCTCAGAGTCACCCCTAAGTACCGCAGGCGGCTCAGGTTGCGGAAGGCGGCGGCGGGCAGGTGCGCA GCTGGGTGCGGTTCAGCCACAGCTCCTGCAGGCCCCCATCTCCCCGAAGAGCACCCCCGGGAGCTCTGCCAGCG GGTTCTCGAACAGAGTCAACAGAGTCAGATTGTGCGAATGAAGAAAGAGCGCAGAGGGGAGAAACGCAAGGTGGT TTCTCGAAAGCGTCAAAGAACTGAGGTTTGGGAGCCGGTCGAAGGCCCCGGGTGCGATGGAACGGATGTGATTTC GGTGGAACTGCAGCTCCGTCAGGGCGCCCAGGCTGTTCAACAGCCCCGAATCCAGAGACACAAGGCGGTTCGAGT GGAGCAGAAGTCTCTCGAGCTTAGCCTGTGCTCCAAGCAACCCCTTGGGCAGGTGGGTCAGGTTGTTTCCCGATAA ATCCAACAACTTCAGGTTCTCCAGATTCGTGAAGAGACTGGCAGGAAGGAAATCGAGCTGATTCTGGTTCAGAGCG AGCTCCTGCAGGTTAACCAGTTTCTGAAACATGTTTTGGTCAATGCCCCTTAGCGCATTGTGGTCCAAAAACAACTGC TCCAGGAGCACCATCTTATCCAGCAGCGCACCTGGAAGATGCGTGATTTTGTTGCGCGACAGCCTCAGGGTTTTCAG TTTTATCAGGTCACTGAAGGTGCCGGGGGCAACGGCGGAAATGTGGCTGTCGGAGATCATGAGGCGCTGCAGGAC GGTCATGCCGCTGAAGCTCTGGCTCTGCAGGACGCCGCGGCCCATTCCGAAGAGCAGGATGTGCGTGAGGTTGGT GGGCAGGCCTAGCGCGGAGATGCGCGCCACGTCGCCCCCCGAGCACTGCGCGGCGTCCCGGAAGACACACTTGC AAGCTGGCGGACAGGGGAAGGGCTGGGCGCGCAGAAGCCCGAGCACCGCGCACAGTAGAGTCCCCCTCAGCATG TCTGAAAAAGCAACCGTGGGAGTGTGGTCAACACACAGGAGCGTTCGCGCCTGTACTGAACCCTGGGATCC |

| |
|---|
| SEQ ID NO:6 DNA sequence of human GSTP1 |
| TGAAGCGGGTGTGCAAGCTCCGGATCGCAGCGGTCTTAGGGAATTTCCCCCGCGATGTCCCGGCGCGCCAGTT CGCTGCGCACACTTCGCTGCGGTCCTCTTCCTGCTGTCTGTTTACTCCCTAGGCCCCGCTGGGGACCTGGGAAAGA GGGAAAGGCTTCCCCGGCCAGCTGCGCGGCGACTCCGGGGACTCCAGGGCGCCCCTCTGCGGCCGACGCCCGG GGTGCAGCGGCCGCCGGGGCTGGGGCCGGCGGGAGTCCGCGGACCCTCCAGAAGAGCGGCCGGCGCCGTGA CTCAGCACTGGGGCGGAGCGGGGCGGACCACCCTTATAAGGCTCGGAGGCCGCGAGGCCTTCGCTGGAGTTTC GCCGCCGCAGTCTTCGCCACCAGTGAGTACGCGCGGCCCGCGTCCCCGGGGATGGGGCTCAGAGCTCCCAGCAT GGGGCCAACCCGCAGCATCAGGCCCGGGCTCCCGGCAGGGCTCCTCGCCCACCTCGAGACCCGGGACGGGGGC CTAGGGGACCCAGGACGTCCCCAGTGCCGTTAGCGGCTTTCAGGGGGCCCGGAGCGCCTCGGGGAGGATGGGA CCCCGGGGGCGGGGAGGGGGGGCAGACTGCGCTCACCGCGCCTTGGCATCCTCCCCCGGGCTCCAGCAAACTTT TCTTTGTTCGCTGCAGTGCCGCCCTACACCGTGGTCTATTTCCCAGTTCGAGGTAGGAGCATGTG |

| |
|---|
| SEQ ID NO:7 DNA sequence of human PITX2 |
| ACTCCGTGTGGGGAGTGACGTGACGTCAGCAGAGATTCCACCAAACTCCACTGCACAGTGGCGCGGGCGGCCG GCCGAGCCCGGCTGCGCGGCTGGCGATCCAGGAGCGAGCACAGCGCCCGGGCGAGCGCCGGGGGGAGCGAGC AGGGGCGACGAGAAACGAGGCAGGGGAGGGAAGCAGATGCCAGCGGGCCGAAGAGTCGGGAGCCGGAGCCGG GAGAGCGAAAGGAGAGGGGACCTGGCGGGGCACTTAGGAGCCAACCGAGGAGCAGGAGCACGGACTCCCACTG TGGAAAGGAGGACCAGAAGGGAGGATGGGATGGAAGAGAAGAAAAAGCAATCTGCGCCAACCCGGCAGCCCTAAT AAATCAAAGGGGGAGCGCCAGGGCAGCGGGGAGACAGAAACGTACTTTTGGGGAGCAAATCAGGACGGGCTGGG AGGAAGCGACAGGGAAAGTGGCCCAAGAGACGGAACAAAGGACAATGTTCATGGGGTTGTTTGGGACGAGGCGT GTGGAGTGTGGGTGTGAGCGTGCGTGTGTGACCTT |

| |
|---|
| SEQ ID NO:8 DNA sequence of human PITX2 |
| TGGAGGAGTCCAGCGACCGGGGCTGACCGGAGCCAGAACCGAAGCCATGGCTAACGGCTGGGGATGGTGACAG GAAGATGAGGAGACGGCCGACAGCTTGGTCCCGCTGCTCGGTGCTCCAAGTGAAGCGGGCCTTTCATGCAGTTC ATGGACGAGGGAGCGCGACGCTCTACTAGTCCTTGGCTACTGCCCCGCCGAGCCCCCGTAGCCGCCGCTGCCCGC TCCGGGTCGCGCTCTAGGCGCGGAGTTTCCCCGCTGCGGGGAGAGCCAGGGGACGCAACCCCCGCCGAGTTCTC AAGCCAAGCTGCCCCCGTCTCCTCCGGAAGGCTCAAGCGAAAAGTCCGGAGACGGAAAGTCAGCGGGCAAACG AAGACATGGGATGTGGGCAGAAGGGCACCACTCAGAGCGTCTTTAGGGAGCAGGCTTCCAAGCTCCAAAGCGAAA CAAGAGTGGGCAAAGACCCCCTTCTTCTCTCCCTCCCTCCCCCAAGAACCCCTCCAATAAGGAAAGCTAACGCCGAC CGCGCTCTGCCCGCCCCCCCCCACGCG |

| |
|---|
| SEQ ID NO:9 DNA sequence of human RARB |
| ACAGACAGAAAGGCGCACAGAGGAATTTAAAGTGTGGGCTGGGGGCGAGGCGGTGGCGGGAGGCGAGCGGG CGCAGGCGGAACACCGTTTTCCAAGCTAAGCCGCCGCAAATAAAAAGGCGTAAAGGAGAGAAGTTGGTGCTCAA CGTGAGCCAGGAGCAGCGTCCCGGCTCCTCCCCTGCTCATTTTAAAAGCACTTCTTGTATTGTTTTTAAGGTGAGAAA TAGGAAAGAAAACGCCGGCTTGTGCGCTCGCTGCCTGCCTCTCTGGCTGTCTGCTTTTGCAGGGCTGCTGGGAGTT TTTAAGCTCTGTGAGAATCCTGGGAGTTGGTGATGTCAGACTAGTTGGGTCATTTGAAGGTTAGCAGCCCGGGTAGG GTTCACCGAAAGTTCACTCGCATATATTAGGCAATTCAATCTTTCATTCTGTGTGACAGAAGTAGTAGGAAGTGAGCT GTTCAGAGGCAGGAGGGTCTATTCTTTGCCAAAGGGGGGACCAGAATTCCCCCATGCGAGCTGTTTGAGGACTGGG ATGCCGAGAACGCGAGCGATCCGAGCAGGGTTTGTCTGGGCACCGTCGGGGTAGGATCCGGAACGCATTCGGAAG GCTTTTTGCAAGCATTTACTTGGAAGGAGAACTTGGGATCTTTCTGGGAACCCCCCGCCCCGGCTGGATTGGCCGAG CAAGCCTGGAAAATGGTAAATGATCATTTGGATCAATTACAGGCTTTTAGCTGGCTTGTCTGTCATAATTCATGATTCG GGGCTGGGAAAAAGACCAACAGCCTACGTGCCAAAAAAGGGGCAGAGTTTGATGGAGTTGGGTG |

| |
|---|
| SEQ ID NO:10 DNA sequence of human RASSF1A |
| ATCTCCGCGTGGTGCTTTGCGGTCGCCGTCGTTGTGGCCGTCCGGGGTGGGGTGTGAGGAGGGGACGAAGGAGG GAAGGAAGGGCAAGGCGGGGGGGGCTCTGCGAGAGCGCGCCCAGCCCCGCCTTCGGGCCCCACAGTCCCTGCA CCCAGGTTTCCATTGCGCGGCTCTCCTCAGCTCCTTCCCGCCGCCCAGTCTGGATCCTGGGGGAGGCGCTGAAGTC GGGGCCCGCCCTGTGGCCCCGCCCGGCCCGCGCTTGCTAGCGCCCAAAGCCAGCGAAGCACGGGCCCAACCGGG CCATGTCGGGGGAGCCTGAGCTCATTGAGCTGCGGGAGCTGGCACCCGCTGGGCGCGCTGGGAAGGCCGCACC CGGCTGGAGCGTGCCAACGCGCTGCGCATCGCGCGGGGCACCGCGTGCAACCCCACACGGCAGCTGGTCCCTGG CCGTGGCCACCGCTTCCAGCCCGCGGGGCCCGCCACGCACACGTGGTGCGACCTCTGTGGCGACTTCATCTGGGG CGTCGTGCGCAAAGGCCTGCAGTGCGCGCGTGAGTAGTGGCCCCGCGCGCCTACGAGAGCGGAAGGGGCAGCCA AGGGGCAGCGCAGTCGCCGCGGGTCAAGTCGCGGCAGAGGGGGTCGGCGGGGACAGCTCCCGAGGACTAGGTC CGTTACTTTCGCCCCAT |

| |
|---|
| SEQ ID NO:11 DNA sequence of human SOX17 |
| GGCGTCAGGCTCGCAAAGAACAGTTTGGGGTTTAAGAAATCGGCCTAGCGATTCGCGCCAGCGCGCCCGGCCGCC TGGGTCTGCGACTCGGACTCACCCAGCATCTTGCTCAACTCGGCGTTGTGCAGGTCTGGATTCTGCTGCGCCAGCC GCTTGCGCTCGTCCTTAGCCCACACCATGAAAGCGTTCATCGGCCGCCGGATACGGACTCGCCCTTGGCTCGGCC CGCGGCCCCGGCCGGTGCTCCGCTGTTCGCCGGCGCCTCGCCCTTCACCTTCATGTCCCCGATGGGGCTCAGCGA CTCGGCCCAGGGGCAGGGGCCCAGCCCGGCCATCACCGCGGGCAGCGCGCTCTGGGTCTGGCTCTGGTCGTCAC TGGCGTATCCCGCATCCGGGCTGCTCATGGCGCTCCAGGCCTGCCCCGCTCCCCTCAACCCTCCGCCGGACGCGC CGCCTCCCCCGACCCGGGGGAGGGGGTGGGGAGTGAGGCACTGAGATGCCCCGAGGGCTGCGCGGGTCTCCCG GCCCGAAGCCGCCGCCCGTGTTCTGGCCTGTCGCGGTCTGGTCTACAGCGTACCCAGGGCCCCCAGCCGGCCTAG |
| TGACACTGCGGGCGCCCCTGGCCGCGGGGCCTTTTCTGCACAGATGTGGCCAATGGAGCGGCGAGGGCGGCC GGTCCCGCGTCGTTAGGCCCACGCCCAGGCCGTGGTCCGAGTCCCACGTCCCAGTCCAACCCCACGCCCGCCCCT TGCCCCTCCCCCGGCCCCGCGCCCAAGGCTACACCTGCCCCCGGGAAAACTAGCCGGAGCTGGGCTCTGGCGCC GCGTCCTCTCCCACCGGGGTTAGGGAGACTCGAAAAGCCGTCTGGGAGGGCTGATTGTACCTTGGAATCGACGT |

The term "biomarker gene or a fragment thereof" is often used herein when referring to the detection of a methylation level, because, in the choice of a template, as long as the length of the template is not less than the length of the region to be amplified, the primer pair used in the PCR amplification reaction does not distinguish between the entire gene or a fragment thereof (in fact, during the DNA extraction and subsequent bisulfite treatment, the gene is usually broken into fragments of different sizes).

In some preferred embodiments, the present invention uses the HeavyMethyl method to measure marker gene methylation. Therefore, in addition to the design of common Taqman primers, blocking primers are further designed. The nucleotide sequence of a blocking primer is designed to be paired with and bind to a template sequence in the region amplified by a corresponding primer pair. In addition, a chemical modification is introduced into a blocking primer at 3'-OH, which prevents the amplification with a DNA polymerase. The chemical modifications are, for example, C3 spacer (C3 Spacer), C6 spacer (C6 Spacer), inverted 3'end , 3' phosphate (3'P), etc. In embodiments of the method of the present invention, the nucleotide sequence of a blocking primer is designed to bind to an unmethylated template (treated with sulfite), but not to a methylated template (treated with sulfite). Therefore, when no methylation occurs in the region corresponding to a blocking primer, it can prevent the corresponding amplification reaction, and thereby improving the specificity of the detection method of the present invention.

In further preferred embodiments of the method of the present invention, it also comprises the use of fluorescent probes to monitor and/or quantify PCR amplification reactions in real time. The fluorescent report group at 5' end of a probe used may be FAM, JOE, TET, HEX, Cy3, Texas Red, Rox, or Cy5; the quenching group at the 3' end is BHQ1, BHQ2, BHQ3, TAMRA, DABCYL, or MGB.

The detection of the methylation levels of the biomarker gene(s) in the method of the present invention includes detecting whether there is/are methylation(s) in the biomarker gene, and quantitative and qualitative detection of the methylation(s).

The biological sample is selected from fluids or tissues extracted form the subject, and includes blood, serum, plasma, breast duct fluid, lymph, cerebrospinal fluid, ascite, urine, tissue biopsy, etc., preferably plasma, serum and breast duct fluid.

In the method of the present invention, the age of the subject can also be considered to predict the breast cancer status in the subject.

In some embodiments, the method of the present invention further comprises the step of providing a written report or an electronic report on the breast cancer prediction, and optionally, the report comprises a prediction about the presence or not or likelihood of a breast cancer in the subject, or about the risk gradation of a breast cancer in the subject.

In some embodiments, the method of the present invention also comprises establishing a report for a physician on the relative methylation levels of biomarker gene(s), and transmitting such report by post, fax, mailbox, etc. In one embodiment, a data stream containing the report of methylation levels of biomarker gene(s) is transmitted through the internet.

In some embodiments, a statistical method is used to construct a diagnostic model based on the methylation levels of the biomarker gene(s). The statistical method is selected from the following methods: multiple linear regression, lookup table, decision tree, support vector machine, Probit regression, logistic regression, cluster analysis, neighborhood analysis, genetic algorithm, Bayesian and non-Bayesian methods, etc.

In other embodiments, a prediction or diagnostic model based on the methylation levels of the biomarker gene(s) is provided. The model may be in the form of software code, a computer-readable format, or a written description for evaluating the relative methylation levels of the biomarker gene(s).

New and important additional information, which assists the physician in grading the risk of a patient suffering from a breast cancer and planning the diagnostic steps to be taken next, can be obtained by using the method of the present invention. The method provided herein can similarly be used to assess the risk of a breast cancer in an asymptomatic high-risk patient, and as a screening tool for the general population. It is contemplated that the method of the present invention can be used by a clinician as part of a comprehensive assessment of other predictive and diagnostic indicators.

The method of the present invention can be used to evaluate the therapeutic efficacies of existing chemotherapeutic agents, candidate chemotherapeutic agents and other types of cancer treatments. For example, biological samples can be taken from a subject before or after a treatment or during a treatment of the subject, and the methylation levels of the biomarker gene(s) can be detected as described above. The detection results are used to identify changes in the cancer status in the subject so as to determine the therapeutic efficacy.

The method of the invention can also be used to identify whether a subject is potentially developing a cancer. Relative methylation levels of the biomarker gene(s) in biological samples taken from a subject over time are detected, and the changes in the methylation levels of the biomarkers that point to the characteristics of a cancer are interpreted as a progress toward the cancer.

The combination of the biomarker genes provides a sensitive, specific and accurate means for predicting the presence of a breast cancer or detecting a breast cancer in different stages of the breast cancer progression. Evaluation of the methylation levels in the biological sample may also be correlated with the presence of a pre-malignant or pre-clinical disorder in a patient. Therefore, the disclosed method can be used to predict or detect the presence of a breast cancer in a sample, the stage of a breast cancer, the subtype of a breast cancer, the benignity or malignancy of a breast cancer, the possibility of metastasis of a breast cancer, the histological type of a neoplasm associated with a breast cancer, the painlessness or aggressiveness of a cancer, and other breast cancer characteristics related to the prevention, diagnosis, characterization, and treatment of a breast cancer in a patient.

The method of the present invention can also be used to evaluate the effectiveness of candidate drugs to inhibit breast cancer, evaluate the efficacy of breast cancer therapy, monitor the progress of breast cancer, select agents or therapies to inhibit breast cancer, monitor the treatment of breast cancer patients, monitor the inhibition status of breast cancer in patients, and test the methylation levels of biomarker genes in animals after exposure to test compounds to assess the carcinogenic potential of the test compounds.

The present invention also provides a kit for detecting the breast cancer status. In some embodiments, the kit may include a DNA extraction reagent and a bisulfite reagent. The DNA extraction reagent may include a lysis buffer, a binding buffer, a washing buffer, and an elution buffer. The lysis buffer is usually composed of a protein denaturant, a detergent, a pH buffering agent and a nuclease inhibitor. The binding buffer is usually composed of a protein denaturant and a pH buffer agent. The washing buffer is divided into washing buffer A and washing buffer B: washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is usually composed of a nuclease inhibitor and a pH buffering agent. The protein denaturant is selected from one or more of guanidine isothiocyanate, guanidine hydrochloride and urea; the detergent is selected from one or more of Tween20, IGEPAL CA-630, Triton X-100, NP-40 and SDS; the pH buffering agent is selected from one or more of Tris, boric acid, phosphate, MES and HEPES; the nuclease inhibitor is selected from one or more of EDTA, EGTA and DEPC. The bisulfite reagents include a bisulfite buffer and a protective buffer, in which the bisulfite salt is selected from one or more of sodium metabisulphite, sodium sulfite, sodium bisulfite, ammonium bisulfite and ammonium sulfite; the protection buffer is composed of an oxygen radical scavenger, and the oxygen radical scavenger is selected from one or more of hydroquinone, vitamin E, vitamin E derivatives, gallic acid, Trolox, trihydroxybenzoic acid and trihydroxybenzoic acid derivatives.

The kit of the present invention comprises a primer pair or primer pairs for methylation-specific PCR amplification reaction(s) for one or more of APC, BRCA1, CCND2, CST6, GP5, GSTP1, PITX2, RARB, RASSF1A and SOX17 gene. These primer pairs respectively detect the methylation of at least one nucleotide sequence in the nucleotide sequence of a target region of the corresponding gene.

The kit of the present invention may further comprise blocking primers and probes used in combination with the above-mentioned primer pairs (these blocking primers and probes are described above and below).

In certain embodiments, the kit may further comprise an instruction for using the kit to extract DNA from a biological sample and treating the DNA with the bisulfite reagent. In other embodiments, the kit further comprises an instruction for using the reagents in the kit to measure a biomarker level in the subject. In still other embodiments, the kit comprises an instruction for using the kit to determine the breast cancer status in a subject.

The present invention also protects the method for detecting the methylation levels of the biomarker genes or fragments thereof with the kit. The method comprises the steps: extracting DNA in a biological sample by using the DNA extraction reagents, treating the extracted DNA with the bisulfite reagents, and using the treated DNA as a template to detect the methylation levels of the biomarker genes with the provided primer pairs.

The measurement method for the methylation level of a biomarker gene may be selected from one or more of the following methods: real-time fluorescent PCR, digital PCR, bisulfite sequencing, methylation-specific PCR, restriction enzyme analysis, high-resolution dissolution curve technology, gene chip technology and time-of-flight mass spectrometry.

The present invention is further described by the following examples.

### Example 1: DNA extraction

The DNA extraction reagent is composed of a lysis buffer, a binding buffer, a washing buffer, and an elution buffer. The lysis buffer is composed of a protein denaturant, a detergent, a pH buffering agent and a nuclease inhibitor. The binding buffer is composed of a protein denaturant and a pH buffering agent. The washing buffer is divided into washing buffer A and washing buffer B. Washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is composed of a nuclease inhibitor and a pH buffering agent. The protein denaturant is guanidine hydrochloride; the detergent is Tween20; the pH buffering agent is Tris-HCl; and the nuclease inhibitor is EDTA.

In this example, a plasma sample of a breast cancer patient is taken as an example to extract plasma DNA. The extraction method comprises the following steps:
(1) provide 1 mL plasma, add the same volume of the lysis buffer, then add proteinase K and Carrier RNA to achieve a final concentration of 100 mg/L and 1 µg/mL, mix by shaking, and incubate at 55°C for 30 min;
(2) add 100µL magnetic beads (purchased from Life technologies, catalog No: 37002D), and incubate for 1 hour with shaking;
(3) adsorbe the magnetic beads with a magnetic separator, and discard the supernatant solution;
(4) add 1mL of the washing buffer A to resuspend the magnetic beads and wash for 1minute with shaking;
(5) adsorb the magnetic beads with the magnetic separator and discard the supernatant;
(6) add 1mL of the washing buffer B to resuspend the magnetic beads and wash for 1minute with shaking;
(7) adsorb the magnetic beads with the magnetic separator and discard the supernatant solution;
(8) quickly centrifuge at 10,000 rpm for 1 minute, absorb the magnetic beads with the magnetic separator, and remove the residual supernatant solution;
(9) place the centrifuge tube loaded with the magnetic beads on a 55°C metal bath, and dry it for 10 minutes, with the lid open;
(10) add 100 µL of the elution buffer to resuspend the magnetic beads, place it on a 65°C metal bath, and elute for 10 minutes with shaking;
(11) adsorb the magnetic beads with the magnetic separator, take out the buffer containing the target DNA, quantify the DNA, and make a mark;
(12) store the eluted DNA in a refrigerator at 4°C for later use, or in a refrigerator at -20°C for long-term storage.

### Example 2: treatment of DNA with bisulfite

Treatment of DNA with bisulfite is to treat the extracted DNA sample with the bisulfite reagent. The bisulfite reagent is composed of a bisulfite buffer and a protection buffer. The bisulfite buffer is a mixed liquid of sodium bisulfite and water; the protective buffer is a mixed liquid of oxygen radical scavenger hydroquinone and water.

The DNA extracted in Example 1 is used as the processing object in this Example, and the DNA is treated with bisulfite. It comprise:
(1) prepare the bisulfite buffer: weigh 1 g of sodium bisulfite powder, and add water to it to obtain 3 M buffer solution;
(2) prepare the protection buffer: weigh 1 g of hydroquinone reagent, and add water to it to obtain 0.5M protection buffer;
(3) mix together 100 µL of the DNA solution, 200 µl of the bisulfite buffer and 50 µl of the protection solution, and mix by shaking;
(4) thermal treatment: 95°C for 5 minutes, 80°C for 60 minutes, and 4°C for 10 minutes;
(5) add 1 mL of the DNA binding buffer to the bisulfite-treated DNA solution, add 50 mL magnetic beads, and incubate for 1 hour with shaking;
(6) adsorbe the magnetic beads with a magnetic separator, and discard the supernatant solution;
(7) add 0.5mL of the washing buffer A to resuspend the magnetic beads and wash for 1minute with shaking;
(8) adsorbe the magnetic beads with the magnetic separator, and discard the supernatant;
(9) add 0.5mL of the washing buffer B to resuspend the magnetic beads and wash for 1minute with shaking;
(10) adsorbe the magnetic beads with the magnetic separator, and discard the supernatant;
(11) quickly centrifuge at 10,000 rpm for 1 minute, absorb the magnetic beads with the magnetic separator, and remove the residual supernatant solution;
(12) place the centrifuge tube loaded with the magnetic beads on a 55°C metal bath, and dry it for 10 min, with the lid open;
(13) add 50 µL of the elution buffer to resuspend the magnetic beads, place it on a 65°C metal bath, and elute for 10 minutes with shaking;
(14) adsorb the magnetic beads with the magnetic separator, take out the buffer containing the target DNA, quantify the DNA, and make a mark.

### Example 3: Real-time fluorescent PCR detection of DNA methylation and verification of primer sets

In this example, a real-time fluorescent PCR was used as an example to detect the methylation levels of biomarker genes. The genes to be detected were APC, BRCA1, CCND2, CST6, GP5, GSTP1, PITX2, RARB, RASSF1A and SOX17 genes, and the internal reference gene was ACTB. In this example, the bisulfite-treated DNA of Example 2 was used as a template for real-time fluorescent PCR amplification. The DNA samples to be detected, a negative quality control product, a positive quality control product and no template controls were all detected in three replicates. The negative quality control product and the positive quality control product were respectively prepared as follows: take 400 µL of human leukocyte DNA with a concentration of 10 ng/µL and add it to TE buffer solution containing 1% BSA, mix, and dilute the solution to 200 mL to obtain a negative control substance with a concentration of 0.02 ng/µL; take 384 µL of human leukocyte DNA at a concentration of 10 ng/µL and 16 µL of Hela cell DNA at a concentration of 10 ng/µL, add them to TE buffer solution containing 1% BSA, mix, and dilute to 200 mL to obtain a concentration of 0.02 ng/µL, in which the positive DNA content is 4%.

For APC, BRCA1, CCND2, CST6, GP5, GSTP1, PITX2, RARB, RASSF1A and SOX17 genes, multiple sets of primer and probe combinations could be designed. However, the performance of each set of the probe and primer combinations may be different, so they needed to be verified through experiments.

Therefore, we designed a variety of primers and probes for APC, BRCA1, CCND2, CST6, GP5, GSTP1, PITX2, RARB, RASSF1A and SOX17 genes, which were respectively equivalent to, complementary to, or hybridizable to at least 15 consecutive nucleotides of the sequences as set forth in SEQ ID NOs: 1-11 or complementary sequences thereof, and verified the effectiveness of the designed primers and probes with methylated and unmethylated nucleic acid sequences as templates. We selected the following optimal primer sets and a primer set for the internal reference gene ACTB through real-time fluorescence PCR amplification results.
APC primer set 1
   primer 1: SEQ ID NO 12: 5'- TCGCGAGGGTATATTTTCGAGG -3'
   primer 2: SEQ ID NO 13: 5'- ATCCGCATCCAACGAATTACAC -3'
   blocking primer: SEQ ID NO 14: 5'- TTGAGGGGTATGGGGTTAGGGTTAGG -C3-3'
   probe: SEQ ID NO 15: 5'-HEX- ACACAAAACCCCGCCCAACCG -BHQ1-3'
APC primer set 2
   primer 1: SEQ ID NO 16: 5'- GGCGTTTTTTATTTTCGTCGGG -3'
   primer 2: SEQ ID NO 17: 5'- TACCCCATTTCCGAATCCGAC -3'
   blocking primer: SEQ ID NO 18: 5'- GTTTTTTATTTTTGTTGGGAGTTTGTTGATTG -C3-3'
   probe: SEQ ID NO 19: 5'-HEX- TACGCCCACACCCAACCAATCG -BHQ1-3'
BRCA1 primer set 1
   primer 1: SEQ ID NO 20: 5'- TACGTTATTCGGGGGTAGATTGG -3'
   primer 2: SEQ ID NO 21: 5'- CGCGCTTTTCCGTTACCAC -3'
   blocking primer: SEQ ID NO 22: 5'- TGTTATTTGGGGGTAGATTGGGTGG -C3-3'
   probe: SEQ ID NO 23: 5'-Texas Red- AAACCAAACGTCTCTCGAAACTCTAAATTAAC -BHQ2-3'
BRCA1 primer set 2
   primer 1: SEQ ID NO 24: 5'- ACGTCGGTTGGTTATGAGGTTAG -3'
   primer 2: SEQ ID NO 25: 5'- CGAATTCTAACGATTCTCCTACCTC -3'
   blocking primer: SEQ ID NO 26: 5'- TGTTGGTTGGTTATGAGGTTAGGAGTTTT -C3-3'
   probe: SEQ ID NO 27: 5'-Texas Red- AAACGAAATTTCACCACGTTAATCAAACT -BHQ2-3'
BRCA1 primer set 3
   primer 1: SEQ ID NO 28 5'- GCGTGGGAGAGTGGATTTTCG -3'
   primer 2: SEQ ID NO 29: 5'- TCCTCAACGCTTCCCTCGC -3'
   blocking primer: SEQ ID NO 30: 5'- TGTGGGAGAGTGGATTTTTGAAGTTGATAG -C3-3'
   probe: SEQ ID NO 31: 5'-Texas Red- AAATTCCGCCCCTACCCCCCG -BHQ2-3'
CCND2 primer set 1
   primer 1: SEQ ID NO 32 5'- TACGGTTTATTTAGTTCGCGTTTTAT -3'
   primer 2: SEQ ID NO 33: 5'- CCAACGCCCTAATATACTAACCAAACT -3'
   blocking primer: SEQ ID NO 34: 5'- TTTAGTTTGTGTTTTATTTTGTTTTGTTGGTTTTT -C3-3'
   probe: SEQ ID NO 35: 5'-Texas Red- AAACCCGAACAAAAACGCGAAAAAC -BHQ2-3'
CCND2 primer set 2
   primer 1: SEQ ID NO 36: 5'- TGCGTTAGAGTACGTGTTAGGGTC -3'
   primer 2: SEQ ID NO 37: 5'- CGAATAAAAAATTAAATCCGACTCC -3'
   blocking primer: SEQ ID NO 38: 5'- GAGTATGTGTTAGGGTTGATTGTGTTGGT -C3-3'
   probe: SEQ ID NO 39: 5'-Texas Red- AACCGACTACGATAAAATCGCCGCC -BHQ2-3'
CCND2 primer set 3
   primer 1: SEQ ID NO 40: 5'- GTTGTATCGGTGTGGTTACGTTTAGC -3'
   primer 2: SEQ ID NO 41: 5'- TCTAAAAACTCTTCGAACGCCGT -3'
   blocking primer: SEQ ID NO 42: 5'- ATTGGTGTGGTTATGTTTAGTGTAGATATTTTGG -C3-3'
   probe: SEQ ID NO 43: 5'-Texas Red- TCGCCCCTACATCTACTAACAAACCGC -BHQ2-3'
CST6 primer set 1
   primer 1: SEQ ID NO 44: 5'- GAAGCGTTTTGGTACGCGG -3'
   primer 2: SEQ ID NO 45: 5'- ATACGCCCCCGCGACCT -3'
   blocking primer: SEQ ID NO 46: 5'- TGTTTTGGTATGTGGGTGTGTGTTGTTT -C3-3'
   probe: SEQ ID NO 47: 5'-FAM- TACGAAATTCAAAAAACCCAAAAAACCG -BHQ1-3'
CST6 primer set 2
   primer 1: SEQ ID NO 48: 5'- TTTTTCGTTGGCGTTGGGT -3'
   primer 2: SEQ ID NO 49: 5'- ACACCTACGAATCGTCGAACGAC -3'
   blocking primer: SEQ ID NO 50: 5'- TGTTGGTGTTGGGTTTGGTTTTGGT -C3-3'
   probe: SEQ ID NO 51: 5'-FAM- TCGCGTAACAACGCCAAAAAACAAAAT -BHQ1-3'
GP5 primer set 1
   primer 1: SEQ ID NO 52: 5'- TCGAAGAGTATTTTCGGGAGTTTTG -3'
   primer 2: SEQ ID NO 53: 5'- TTAACGCTTTCGAAAAACCACC -3'
   blocking primer: SEQ ID NO 54: 5'- TTTTGGGAGTTTTGTTAGTGGGTTTTTG -C3-3'
   probe: SEQ ID NO 55: 5'-Texas Red- TACGCTCTTTCTTCATTCGCACAATCTAAC -BHQ2-3'
GP5 primer set 2
   primer 1: SEQ ID NO 56: 5'- TAGCGTATTTGGAAGATGCGTGAT -3'
   primer 2: SEQ ID NO 57: 5'- TAACCGTCCTACAACGCCTCATAAT -3'
   blocking primer: SEQ ID NO 58: 5'- GATGTGTGATTTTGTTGTGTGATAGTTTTAGGGT -C3-3'
   probe: SEQ ID NO 59: 5'-Texas Red- TACCCCCGACACCTTCAATAACCTAATAAAAC -BHQ2-3'
GP5 primer set 3
   primer 1: SEQ ID NO 60: 5'- TTGGCGGATAGGGGAAGGGT -3'
   primer 2: SEQ ID NO 61: 5'- ACGCGAACGCTCCTATATATTAACC -3'
   blocking primer: SEQ ID NO 62: 5'- TGGATAGGGGAAGGGTTGGGTGTGT -C3-3'
   probe: SEQ ID NO 63: 5'-Texas Red- TCTACTATACGCGATACTCGAACTTCTACGCG -BHQ2-3'
GSTP1 primer set 1
   primer 1: SEQ ID NO 64: 5'- TTTCGGTTAGTTGCGCGG -3'
   primer 2: SEQ ID NO 65: 5'- CTAAAAAATCCCGCGAACTCC -3'
   blocking primer: SEQ ID NO 66: 5'- TTGTGTGGTGATTTTGGGGATTTTAG -C3-3'
   probe: SEQ ID NO 67: 5'-JOE- ACCGCAAAAAAACGCCCTAAAATC -BHQ1-3'
GSTP1 primer set 2
   primer 1: SEQ ID NO 68: 5'- TCGTTGGAGTTTCGTCGTCGT -3'
   primer 2: SEQ ID NO 69: 5'- AACCCCCGTCCCGAATCT -3'
   blocking primer: SEQ ID NO 70 5'- GGAGTTTTGTTGTTGTAGTTTTTGTTATTAGTGAG -C3-3'
   probe: SEQ ID NO 71: 5'-JOE- ACGCGAACCGCGCGTACTCACT -BHQ1-3'
PITX2 primer set 1
   primer 1: SEQ ID NO 72: 5'- TTCGTGTGGGGAGTGACGTGAC -3'
   primer 2: SEQ ID NO 73: 5'- TCGTTTCTCGTCGCCCCTACT -3'
   blocking primer: SEQ ID NO 74 5'- GTGGGGAGTGATGTGATGTTAGTAGAGATTTTATT -C3-3'
   probe: SEQ ID NO 75: 5'-HEX- ACCGCCCGCGCGCCACTATAC -BHQ1-3'
PITX2 primer set 2
   primer 1: SEQ ID NO 76: 5'- CGGAGTCGGGAGAGCGAAAG -3'
   primer 2: SEQ ID NO 77: 5'- TAAAACTACCGAATTAACGCAAATTACT -3'
   blocking primer: SEQ ID NO 78: 5'- TTGGGAGAGTGAAAGGAGAGGGGATT -C3-3'
   probe: SEQ ID NO 79: 5'-HEX- TCCTCGATTAACTCCTAAATACCCCGCC -BHQ1-3'
PITX2 primer set 3
   primer 1: SEQ ID NO 80: 5'- TGAGGAGACGGTCGATAGTTTGG -3'
   primer 2: SEQ ID NO 81: 5'- ACGAACAACGACGACTACGAAAACT -3'
   blocking primer: SEQ ID NO 82: 5'- GAGATGGTTGATAGTTTGGTTTTTGTTGTTTG -C3-3'
   probe: SEQ ID NO 83: 5'-HEX- TAAAAAACCCGCTTCACTTAAAACACCG -BHQ1-3'
PITX2 primer set 4
   primer 1: SEQ ID NO 84: 5'- GAGACGGAAAGTTAGCGGGTAAAC -3'
   primer 2: SEQ ID NO 85: 5'- CAAAACGCGATCGACGTTAACT -3'
   blocking primer: SEQ ID NO 86: 5'- ATGGAAAGTTAGTGGGTAAATGAAGATATGG -C3-3'
   probe: SEQ ID NO 87: 5'-HEX- TACTCCCTAAAAACGCTCTAAATAATACCCTTCT -BHQ1-3'
RARB primer set 1
   primer 1: SEQ ID NO 88: 5'- GCGTATAGAGGAATTTAAAGTGTGG -3'
   primer 2: SEQ ID NO 89: 5'- ACGCCTTTTTATTTACGACGACTTAAC -3'
   blocking primer: SEQ ID NO 90: 5'- TTATTTACAACAACTTAACTTAAAAAACAATATTCCACC -C3-3'
   probe: SEQ ID NO 91: 5'-FAM- TATTCCGCCTACGCCCGCTCG -BHQ1-3'
RARB primer set 2
   primer 1: SEQ ID NO 92: 5'- GAATTTTTTTATGCGAGTTGTTTGAGG -3'
   primer 2: SEQ ID NO 93: 5'- TTCCGAATACGTTCCGAATCCTACC -3'
   blocking primer: SEQ ID NO 94: 5'- TTATGTGAGTTGTTTGAGGATTGGGATGTTGAG -C3-3'
   probe: SEQ ID NO 95: 5'-FAM- AACAAACCCTACTCGAATCGCTCGCG -BHQ1-3'
RARB primer set 3
   primer 1: SEQ ID NO 96: 5'- TGGGAATTTTTCGTTTCGGTT -3'
   primer 2: SEQ ID NO 97: 5'- ACACGTAAACTATTAATCTTTTTCCCAAC -3'
   blocking primer: SEQ ID NO 98: 5'- CATAAACTATTAATCTTTTTCCCAACCCCAAATC -C3-3'
   probe: SEQ ID NO 99: 5'-FAM- TCATTTACCATTTTCCAAACTTACTCGACC -BHQ1-3'
RASSF1A primer set 1
   primer 1: SEQ ID NO 100: 5'- AAGGAAGGGTAAGGCG -3'
   primer 2: SEQ ID NO 101: 5'- CGAACGAAACCACAAAAC -3
   blocking primer: SEQ ID NO 102: 5'- GTGGGGGGGGTTTTGTGAG -C3-3'
   probe: SEQ ID NO 103: 5'-FAM- AACCCCGACTTCAACGCCTC -BHQ1-3'
RASSF1A primer set 2
   primer 1: SEQ ID NO 104: 5'- TTGTTAGCGTTTAAAGTTAGC -3'
   primer 2: SEQ ID NO 105: 5'- TAAAATTACACGCGATACCC -3
   blocking primer: SEQ ID NO 106: 5'- AGTGTTTAAAGTTAGTGAAGTATGGGTT -C3-3'
   probe: SEQ ID NO 107: 5'-FAM- ACACGCTCCAACCGAATACGACC -BHQ1-3'
RASSF1A primer set 3
   primer 1: SEQ ID NO 108: 5'- TCGTTTTTAGTTCGCGG -3'
   primer 2: SEQ ID NO 109: 5'- TTAACTACCCCTTCCGCT -3
   blocking primer: SEQ ID NO 110: 5'- TAGTTTGTGGGGTTTGTTATGTATATGT -C3-3'
   probe: SEQ ID NO 111: 5'-FAM- ACAAACCTTTACGCACGACGCCC -BHQ1-3'
SOX17 primer set 1
   primer 1: SEQ ID NO 112: 5' TTCGGTCGTTTGGGTTTGC -3'
   primer 2: SEQ ID NO 113: 5'- CAAAAACGAATCCCGTATCCG -3'
   blocking primer: SEQ ID NO 114: 5'- TTGTTTGGGTTTGTGATTTGGATTTATTTAG -C3-3'
   probe: SEQ ID NO 115: 5'-HEX- TCCAAACCTACACAACGCCGAATTAAAC -BHQ1-3'
SOX17 primer set 2
   primer 1: SEQ ID NO 116: 5'- TTAGTTCGGTTATTATCGCGGGT -3'
   primer 2: SEQ ID NO 117: 5'- CGCGCAACCCTCGAAACAT -3'
   blocking primer: SEQ ID NO 118: 5'- TGGTTATTATTGTGGGTAGTGTGTTTTGGGT -C3-3'
   probe: SEQ ID NO 119: 5'-HEX- TACGCCAATAACGACCAAAACCAAACC -BHQ1-3'
SOX17 primer set 3
   primer 1: SEQ ID NO 120: 5'- TCGCGGTTTGGTTTATAGCGT -3'
   primer 2: SEQ ID NO 121: 5'- TAAAACTCGAACCACGACCTAAACG -3'
   blocking primer: SEQ ID NO 122: 5'- TGGTTTGGTTTATAGTGTATTTAGGGTTTTTAGTTG -C3-3'
   probe: SEQ ID NO 123: 5'-HEX- CGCCCGCAATATCACTAAACCGACT -BHQ1-3'
ACTB primer set
   primer 1: SEQ ID NO 124: 5'-GTGATGGAGGAGGTTTAGTAAGT-3'
   primer 2: SEQ ID NO 125: 5'-CCAATAAAACCTACTCCTCCCTT-3'
   probe: SEQ ID NO 126: 5'-Cy5-ACCACCACCCAACACACAATAACAAACACA-BHQ3-3'

All of the multiple sets of primers and probes could distinguish between methylated and unmethylated templates, and could be used as primers and probes to detect the methylations of APC, BRCA1, CCND2, CST6, GP5, GSTP1, PITX2, RARB, RASSF1A and SOX17 genes, respectively. Although the effectiveness of different primer and probe combinations were slightly different, the above primers and probes were suitable for the detection of methylations of APC, BRCA1, CCND2, CST6, GP5, GSTP1, PITX2, RARB, RASSF1A and SOX17 genes, respectively. Table 1 below showed the detection results of methylated and unmethylated templates (treated with bisulfite) of the above genes with various primer and probe combinations. Obviously, the designed primer and probe combinations were highly specific for the methylated templates.

**Table 1 detection results of the designed primer sets on methylated and unmethylated templates (Ct, mean)**

| | APC- 1 | APC- 2 | BRCA1 -1 | BRCA1 -2 | BRCA1 -3 | CCND2 -1 | CCND2 -2 | CCND2 -3 | CST6 -1 | CST6 -2 |
|---|---|---|---|---|---|---|---|---|---|---|
| **methylated DNA** | 33.23 | 35.86 | 34.76 | 35.25 | 36.23 | 32.16 | 36.78 | 35.29 | 32.78 | 33.92 |
| **unmethylated DNA** | No Ct | No Ct | No Ct | No Ct | No Ct | No Ct | No Ct | No Ct | No Ct | No Ct |
| | | | | | | | | | | |

| | GP5-1 | GP5-2 | GP5-3 | GSTP1 -1 | GSTP1 -2 | PITX2 -1 | PITX2 -2 | PITX2 -3 | PITX2 -4 | RARB -1 |
|---|---|---|---|---|---|---|---|---|---|---|
| **methylated DNA** | 33.23 | 34.98 | 34.47 | 36.29 | 37.89 | 29.38 | 32.29 | 32.89 | 33.65 | 29.81 |
| **unmethylated DNA** | No Ct | No Ct | No Ct | No Ct | No Ct | No Ct | No Ct | No Ct | No Ct | No Ct |
| | | | | | | | | | | |

| | RARB-2 | RARB-3 | RASSF1A- 1 | | RASSF1A- 2 | RASSF1A- 3 | | SOX17- 1 | SOX17 -2 | SOX17 -3 |
|---|---|---|---|---|---|---|---|---|---|---|
| **methylated DNA** | 32.67 | 33.18 | 33.20 | | 34.29 | 33.74 | | 30.28 | 30.65 | 31.29 |
| **unmethylated DNA** | No Ct | No Ct | No Ct | | No Ct | No Ct | | No Ct | No Ct | No Ct |

Furthermore, we used DNAs from different cancer patients and healthy people as templates to further verify the effectiveness of the primer and probe combinations. DNAs in plasma samples from 5 cases of breast cancer, 3 cases of liver cancer, and 5 cases of healthy persons were extracted by using the DNA extraction method of Example 1, and then DNA templates were treated with a bisulfite by using the method of Example 2. Using the above-mentioned multiple primer and probe sets, real-time fluorescent PCR experiments were performed. The Ct values of various marker genes in cancer samples and healthy person samples were measured respectively. The results were shown in Tables 2-4. The detection results listed in Table 2 were from the primer sets of APC, BRCA1, CCND2 and CST6; the detection results listed in Table 3 were from the primer sets of GP5, GSTP1, PITX2 and RARB; and the detection results listed in Table 4 were from the primer sets of RARB, RASSF1A and SOX17.

**Table 2 detection results of the methylation levels of the specified genes in individuals with known breast cancer status (including healthy individuals) with each primer set**

| | APC-1 | APC-2 | BRCA1-1 | BRCA1-2 | BRCA1-3 | CCND2 -1 | CCND2-2 | CND2 -3 | CST6-1 | CST6-2 |
|---|---|---|---|---|---|---|---|---|---|---|
| BC 1 | 32.4 | 34.64 | 33.08 | 34.12 | 35.72 | 34.55 | 35.35 | 34.52 | 33.07 | 34.48 |
| BC 2 | 33.45 | 35.31 | 33.63 | 34.43 | 36.7 | 34.57 | 35.5 | 35.18 | 33.58 | 33.72 |
| BC 3 | 33.48 | 34.67 | 34.84 | 34.91 | 36.3 | 32.13 | 35.03 | 33.2 | 32.08 | 34.92 |
| BC4 | 34.49 | 35.21 | 33.18 | 34.12 | 34.19 | 34.64 | 34.93 | 34.9 | 32.24 | 33.18 |
| 8C 5 | 34.93 | 34.61 | 33.06 | 34.65 | 34.9 | 33.47 | 35.51 | 33.85 | 33.16 | 34.79 |
| HeCa 1 | 42.19 | 44.38 | No Ct | 42.34 | 41.29 | No Ct | 43.29 | 42.19 | 44.32 | 42.15 |
| HeCa 2 | No Ct | No Ct | 42.56 | 44.34 | No Ct | No Ct | 42.57 | No Ct | No Ct | No Ct |
| HeCa 3 | 43.15 | 41.34 | No Ct | 44.23 | 43.67 | No Ct | No Ct | No Ct | 41.38 | No Ct |
| Con 1 | No Ct | No Ct | No Ct | No Ct | No Ct | No Ct | 44.65 | No Ct | No Ct | 44.34 |
| Con 2 | 43.19 | 44.23 | No Ct | No Ct | No Ct | No Ct | No Ct | No Ct | No Ct | No Ct |
| Con 3 | No Ct | No Ct | No Ct | No Ct | 40.56 | No Ct | No Ct | No Ct | No Ct | No Ct |
| Con 4 | No Ct | No Ct | No Ct | 41.29 | No Ct | No Ct | No Ct | No Ct | No Ct | No Ct |
| Con 5 | No Ct | No Ct | 44.23 | No Ct | No Ct | No Ct | No Ct | No Ct | No Ct | No Ct |

| | | | | | | | | | | |
|---|---|---|---|---|---|---|---|---|---|---|
| **abbreviations: BC: breast cancer; HeCa:Liver cancer;Con:healthy** | | | | | | | | | | |

**Table 3 detection results of the methylation levels of the specified genes in individuals with known breast cancer status (including healthy individuals) with each primer set**

| | GP5-1 | GP5-2 | GP5-3 | GSTP1-1 | GSTP1-2 | P1TX2-1 | PITX2-2 | PITX2-3 | PITX2-4 | RARB-1 |
|---|---|---|---|---|---|---|---|---|---|---|
| BC 1 | 33.44 | 35.1 | 34.39 | 37.59 | 34.94 | 31.98 | 35.21 | 33.85 | 34.73 | 31.01 |
| BC 2 | 34.62 | 34.61 | 35.51 | 36.81 | 38.07 | 33.36 | 33.86 | 33.7 | 34.12 | ; 30.86 |
| BC3 | 33.93 | 35.12 | 34.45 | 37.16 | 38.34 | 31.99 | 34.74 | 33.26 | 35.44 | 34.05 |
| BC4 | 34.78 | 35.26 | 35.78 | 38.26 | 36.63 | 32.5 | 35.39 | 34.42 | 34.14 | 31.55 |
| BC 5 | 34.3 | 34.22 | 35.52 | 37.47 | 34.74 | 34.17 | 33.41 | 34.37 | 34.66 | 32.46 |
| HeCa 1 | 42.28 | No Ct | 41.98 | No Ct | No Ct | No Ct | 43.56 | No Ct | No Ct | 41.86 |
| HeCa 2 | No Ct | 44.29 | No Ct | No Ct | No Ct | No Ct | 41.28 | 43.56 | 42.49 | No Ct |
| HeCa 3 | 41.75 | No Ct | No Ct | No Ct | No Ct | 42.39 | No Ct | 42.49 | No Ct | No Ct |
| Con 1 | No Ct | No Ct | No Ct | No Ct | No Ct | No Ct | No Ct | No Ct | No Ct | No Ct |
| Con 2 | 42.87 | No Ct | No Ct | No Ct | 43.98 | No Ct | No Ct | No Ct | 42.19 | No Ct |
| Con 3 | No Ct | No Ct | No Ct | 42.85 | No Ct | No Ct | No Ct | No Ct | No Ct | No Ct |
| Con 4 | No Ct | 43.58 | No Ct | No Ct | No Ct | 44.32 | No Ct | 43.75 | No Ct | No Ct |
| Con 5 | No Ct | No Ct | No Ct | No Ct | No Ct | No Ct | No Ct | 41.28 | 43.26 | No Ct |

| | | | | | | | | | | |
|---|---|---|---|---|---|---|---|---|---|---|
| **abbreviations: BC: breast cancer; HeCa:Liver cancer;Con:healthy** | | | | | | | | | | |

**Table 4 detection results of the methylation levels of the specified genes in individuals with known breast cancer status (including healthy individuals) with each primer set**

| | RARB-2 | RARB-3 | RASSF1A-1 | RASSF1A-2 | RASSF1A -3 | SOX17-1 | SOX17-2 | SOX17-3 |
|---|---|---|---|---|---|---|---|---|
| BC 1 | 34.08 | 33.8 | 32.27 | 33.16 | 32.98 | 34.69 | 31.86 | 32.71 |
| BC2 | 33.88 | 33.27 | 33.44 | 33.87 | 33.73 | 33.59 | 32.35 | 33.66 |
| BC 3 | 34.92 | 33.76 | 33.16 | 34.35 | 35.64 | 32.52 | 32.34 | 31.65 |
| BC4 | 33.01 | 34.99 | 32.54 | 34.74 | 33.87 | 34.47 | 31.01 | 34.39 |
| BC 5 | 33.03 | 34.86 | 34.7 | 35.48 | 33.86 | 33.05 | 34.71 | 35.05 |
| HeCa 1 | No Ct | No Ct | No Ct | 41.87 | No Ct | No Ct | No Ct | No Ct |
| HeCa 2 | 42.36 | No Ct | 42.82 | 42.62 | No Ct | No Ct | 40.87 | No Ct |
| HeCa 3 | 43.98 | 41.46 | No Ct | No Ct | No Ct | No Ct | No Ct | 41.36 |
| Con 1 | No Ct | No Ct | 41.28 | No Ct | No Ct | No Ct | 42.18 | No Ct |
| Con 2 | No Ct | 41.62 | No Ct | 41.29 | No Ct | No Ct | No Ct | No Ct |
| Con 3 | 42.34 | No Ct | No Ct | 42.71 | No Ct | 43.29 | 43.29 | No Ct |
| Con 4 | No Ct | 44.39 | No Ct | No Ct | No Ct | No Ct | No Ct | 41.21 |
| Con 5 | No Ct | No Ct | No Ct | No Ct | No Ct | No Ct | No Ct | No Ct |

| | | | | | | | | |
|---|---|---|---|---|---|---|---|---|
| **abbreviations: BC: breast cancer; HeCa:Liver cancer;Con:healthy** | | | | | | | | |

As can be seen from the above detected Ct values of the methylations of APC, BRCA1, CCND2, CST6, GP5, GSTP1, PITX2, RARB, RASSF1A and SOX17 genes, each of the above primer and probe combination generated a highly specific amplification for methylated DNA of breast cancers, while there were no amplification or the Ct values of the amplifications were greater than 40 for other cancers or the healthy persons. Although the Ct values of the amplifications for breast cancer samples with different combinations of primer pair and probe showed some differences, they were obviously different from those of other cancers and healthy person samples. Therefore, all of the above primer sets were suitable for breast cancer detection.

### Example 4: sensitivity and specificity of the kit for detecting the plasmas of patients with breast cancer or patients with benign disorder

246 samples from patients with pathologically identified breast cancer and 258 samples from patients with pathologically identified benign disorder were used (see Table 5). All of the samples were collected from the Naval General Hospital of People's Liberation Army. The breast cancer samples included all stages and common subtypes of the disease. The breast cancer patients were confirmed by imaging and pathological diagnosis. The sample staging was based on international TNM staging standards, and the sample subtyping was determined according to tissue biopsies and immunohistochemical methods. Benign samples included common types of benign disorders founded in the whole study population. Complete clinical pathology reports were obtained after surgeries, including patient's age, menstruation, race, stage, subtype, and the collection sites were encoded for each sample.

**Table 5 breast cancer stages and other characteristics of the collected samples**

| | breast cancer = stage and subtype | | | | | | benign |
|---|---|---|---|---|---|---|---|
| | 0 | I | II | III | IV | total | |
| **number of samples** | | | | | | | - |
| **breast cancer** | | | | | | | - |
| **infiltrating ductal carcinoma** | 0 | 17 | 22 | 81 | 53 | 173 (70.3) | - |
| **infiltrating lobular carcinoma** | 0 | 5 | 7 | 11 | 15 | 38 (15.4) | - |
| **infiltrating carcinoma of other type** | 0 | 2 | 4 | 3 | 8 | 17 (6.9) | |
| **noninflltrating carcinoma** | 18 | - | - | - | - | 18 (7.3) | - |
| **sum** | 18 | 24 | 33 | 95 | 76 | 246 | - |
| | (7.3) | (9.8) | (13.4) | (38.6) | (30.9) | (100) | - |
| **benign** | | | | | | | |
| **mastitis** | - | - | - | - | - | - | 86 (33.3) |
| **breast cyst** | - | - | - | - | - | - | 56 (21.7) |
| **no abnormalities** | - | - | - | - | - | - | 116 (45.0) |
| **sum** | | | | | | | 258 (100) |
| **ages of the polulation** | | | | | | | |
| **median age (years)** | 46 | 51 | 58 | 61 | 66 | 63 | 50 |
| **age range (years)** | 20-80 | 20-80 | 20-82 | 29-85 | 25-85 | 27-88 | 18-85 |
| **mean age (years)** | 46.5 | 47.6 | 52.3 | 58.9 | 62.3 | 60.5 | 50.1 |
| SD | 11.3 | 13.2 | 9.8 | 10.2 | 10.9 | 9.5 | 8.5 |

DNAs were extracted from the samples by using the DNA extraction method of Example 1, the DNA templates were then treated with bisulfite by using the method of Example 2, and, next, real-time fluorescent PCR experiments were performed with the primer and probe combinations provided in Example 3 (for each biomarker gene, primer set 1 was used) to detect APC, BRCA1, CCND2, CST6, GP5, GSTP1, PITX2, RARB, RASSF1A and SOX17 genes and internal reference gene ACTB, and finally, the Ct values were obtained for each gene from samples of healthy persons and breast cancer patients. As described in Example 3 above, the methylation levels of each gene could be indicated by these Ct values.

Commercially available software packages (IBM SPSS Statistics 24 and MedCalc 11.4.2.0, purchased from IBM and MedCalc, respectively) were used for descriptive statistics of plasma biomarker levels, receiver operating characteristic (ROC) curves and graphical displays. The nonparametric Kruskal-Wallis test (ANOVA) was used, and then a Dunn's multiple comparison post-test was used to determine statistical differences. For all statistical comparisons, p value < 0.05 was considered statistically significant.

The methylation levels of the above 10 marker genes were detected in plasmas from 246 patients with pathologically determined breast cancer and 258 individuals with benign breast disorders by real-time fluorescent PCR assays. To facilitate the determination of the ability of these biomarker genes to distinguish cancers from benign breast disorders with similar symptoms, all samples were obtained from the same clinical population. All samples were collected before any intervention and before the disease status was known. The disease status was then determined by pathological examination of *ex vivo* tissues. A single sample collection protocol was used to collect the plasmas and compliance was monitored. This ensured sample quality and eliminated any possibility of collection, processing and biological bias in the sample set. Normal healthy samples were not used in this study because they are usually more easily distinguishable than benign disorders. These samples showed that the average patient age among individuals with breast cancers (60 years) was higher than that among individuals with benign disorders (50 years), and both increased with the progression of disease stages (Table 5). Overall, the distribution of breast cancer subtypes was similar to that found in all breast cancer cases in the population, with the proportion (80%) of infiltrating carcinomas being larger than that of other breast cancers. The benign controls in the study represented common benign breast diseases, including mastitis, breast cyst, etc.

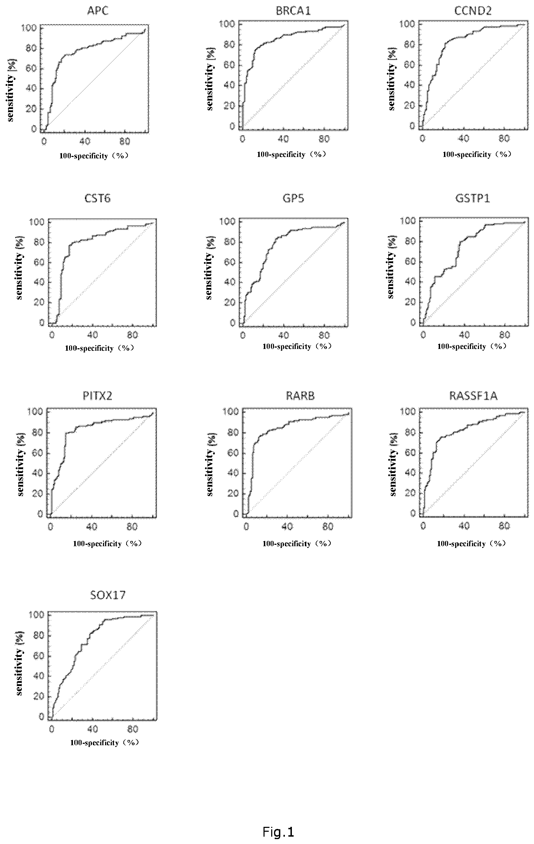

For the detected data of the methylation level of each biomarker, MedCalc 11.4.2.0 software was used to generate a ROC curve and calculate the area under the curve (AUC) value with a 95% confidence interval. Compared with benign breast disorders, the AUCs of the methylation levels of 10 biomarker genes in breast cancer samples were all greater than 0.75 (p value < 0.05), and the AUCs were ranged from 0.75 to 0.86 (see Figure 1 and Table 6).

**Table 6 area under the curves (AUCs) though curve analysis of the receiver operating characteristic (ROC) curves of 10 marker genes**

| **markers** | AUC | **standard error** | 95% Cl |
|---|---|---|---|
| APC | 0.772 | 0.035 | 0.708 - 0.829 |
| BRCA1 | 0.860 | 0.027 | 0.805 - 0.905 |
| CCND2 | 0.839 | 0.028 | 0.781 - 0.887 |
| CST6 | 0.807 | 0.033 | 0.745 - 0.859 |
| GP5 | 0.788 | 0.033 | 0.725 - 0.842 |
| GSTP1 | 0.759 | 0.034 | 0.693 - 0.816 |
| PITX2 | 0.830 | 0.031 | 0.771 - 0.879 |
| RARB | 0.850 | 0.029 | 0.792 - 0.896 |
| RASSF1A | 0.829 | 0.029 | 0.769 - 0.878 |
| SOX17 | 0.777 | 0.033 | 0.713 - 0.833 |

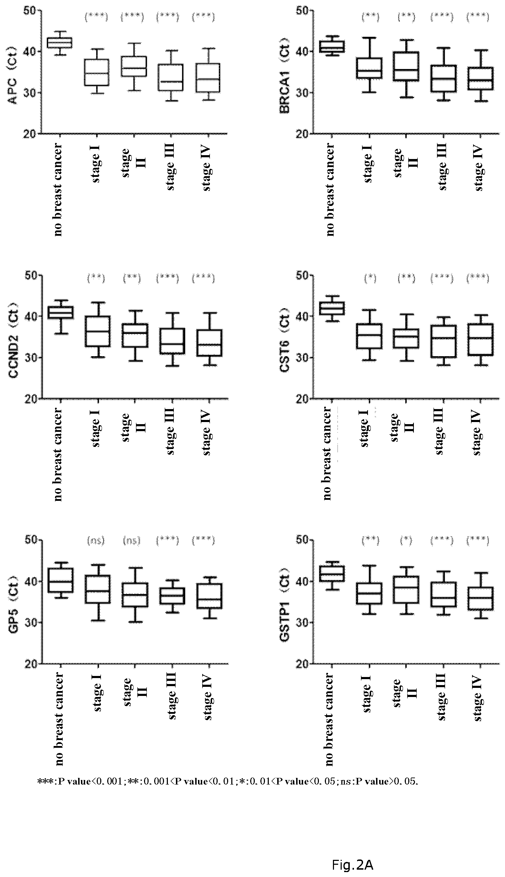

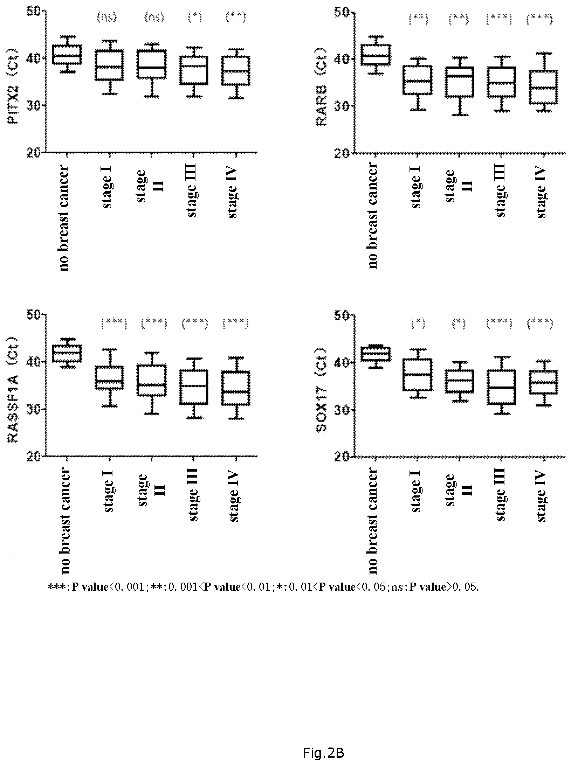

In order to determine whether certain biomarker genes could provide a better distinguishing ability between different stages of breast cancers (especially in early stages), the distinguishing abilities of 10 biomarker genes (Figure 2) in stage I and stage II samples (the most important period for the marker detections) were compared. For stage I samples, APC and/or RASSF1A provided very high distinguishing abilities (p value <0.001), followed by CCND2, RARB, BRCA1 and GSTP1 (p value 0.001 to 0.01) in descending order, and then CST6 and SOX17 (p value 0.01 to 0.05). For GP5 and PITX2, there were no significant differences between stage I cancers and benign disorders (p value > 0.05). For stage II samples, both APC and/or RASSF1A again provided very high distinguishing abilities (p value < 0.001), and then CCND2, RARB, BRCA1 and CST6 (p value 0.001 to 0.01), and then GSTP1 and SOX17 (p value 0.01 to 0.05). There were no significant differences for GP5 and PITX2 (p value > 0.05).

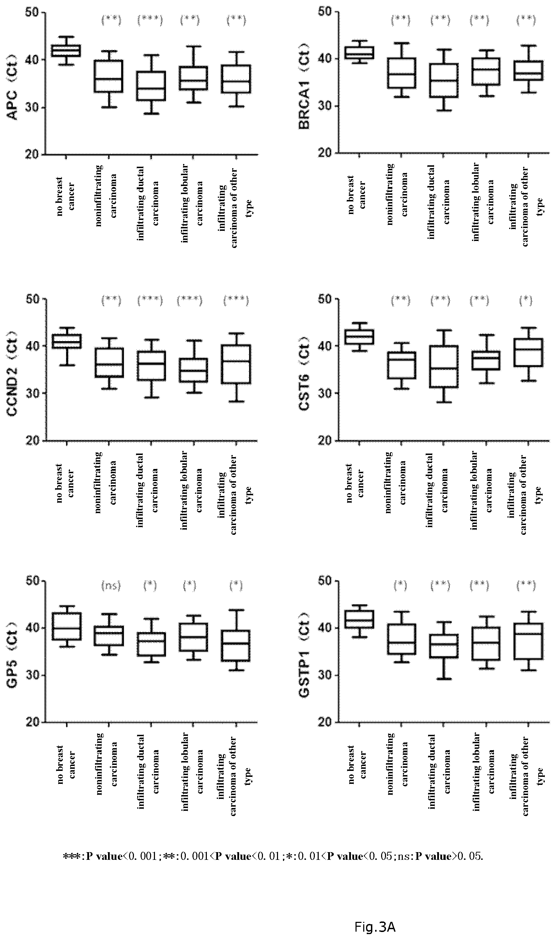

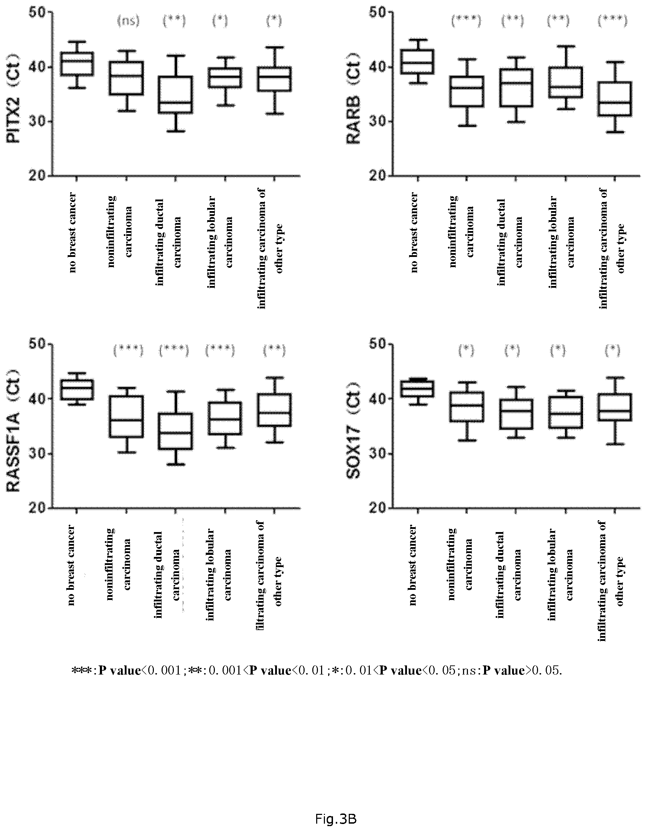

It was also evaluated whether there were statistically significant differences in the methylation levels of the above biomarker genes between samples from benign disorders and various subtypes of breast cancers (Figure 3). For noninfiltrating carcinomas, RARB and RASSF1A provided very high distinguishing abilities (p value < 0.001), followed by CCND2, APC, BRCA1, CST6, GSTP1 and SOX17 (p value 0.001 to 0.05) in descending order. For infiltrating ductal carcinomas, APC, CCND2 and RASSF1A provided very high distinguishing abilities (p value <0.001), followed by RARB, BRCA1, GSTP1, PITX2, CST6, GP5 and SOX17 (p value 0.001 to 0.05) in descending order. For infiltrating lobular carcinomas, CCND2 and RASSF1A provided very high distinguishing abilities (p value <0.001), followed by APC, RARB, BRCA1, GSTP1, CST6, SOX17, GP5 and PITX2 (p value 0.001 to 0.05) in descending order. For other infiltrating carcinomas, CCND2 and RARB provided very high distinguishing abilities (p value <0.001), followed by BRCA1, APC, RASSF1A, GSTP1, CST6 ,SOX17, GP5 and PITX2 (p value 0.001 to 0.05) in descending order.

In terms of simple operation and cost reduction, the detection of the methylation level of a single biomarker gene is better than the detection of the methylation levels of multiple biomarker genes. However, it is obvious that the methylation level of a single biomarker gene may not provide information on the inherent diversity of a complex disease, so it is often necessary to establish a diagnostic model with multiple markers. Multi-marker diagnosis model is established by using statistical analysis methods. The establishment of a diagnosis model with methylated gene markers for the detection of breast cancers is described below by taking a logistic regression model as an example.

The training of the logistic regression model was conducted as follows: dividing the samples into cases and controls, and then optimizing the regression coefficients with IBM SPSS Statistics 24 software. Maximum likelihood of the data was trained with the logistic regression model by using one regression coefficient for each marker and one deviation parameter.

After training, the regression coefficient set defined the logistic regression model. By putting the methylation levels of the biomarkers into the logistic regression equation, those skilled in the art can easily use such diagnostic model to predict the possibility of any new sample to be identified as a case or a control.

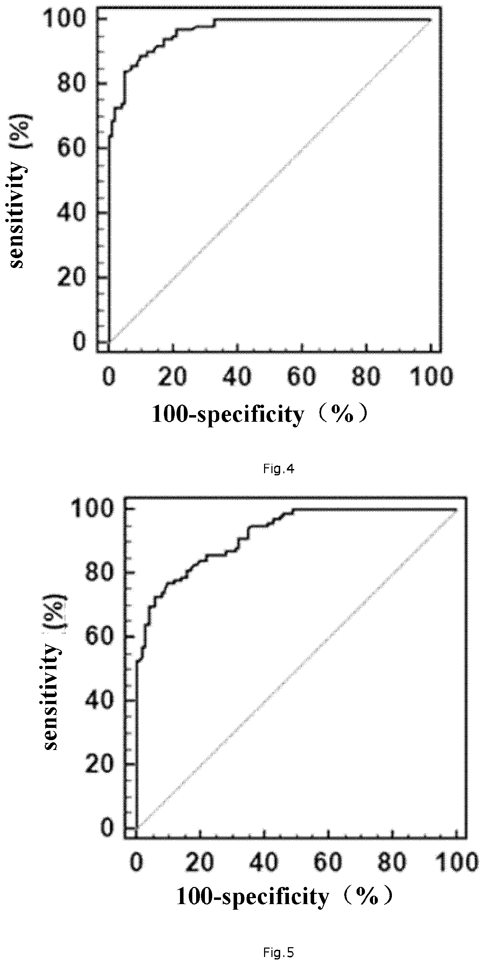

The AUCs of the methylation levels of the above 10 marker genes were all greater than 0.75. Next, we used the logistic regression to combine the 10 marker genes, which generated an AUC of 0.966 (standard error: 0.010; 95% CI: 0.931-0.987; p value < 0.0001) (Figure 4). In order to simplify the monitoring and analysis method, the five markers with larger AUC values were combined and used to establish a logistic regression model. The obtained AUC value was 0.922 (standard error: 0.017; 95% CI: 0.875-0.955; p value: < 0.0001) (Figure 5). Two models were further compared by determining a model's sensitivity at a fixed specificity value and a model's specificity at a fixed sensitivity value (Table 7 and Table 8). For example, it could be selected that, when the sensitivity of the method was greater than about 95%, the sum of its sensitivity and specificity was greater than about 155%; or when the specificity of the method was greater than about 95%, the sum of its sensitivity and specificity was greater than about 160%. Generally, the sensitivity and specificity of a logistic regression model with 10 markers were slightly better than that with 5 markers. However, when the operational analysis procedures and cost were taken into consideration, the combination of the 5 markers may also be a good choice.

**Table 7 sensitivities at important specificity thresholds in logistic regression models of the 5 most characteristic marker genes and of the 10 marker genes**

| **Specificity thresholds(%**) | **sensitivity (%)** | |
|---|---|---|
| | **5 markers** | **10 markers** |
| 80 | 84 | 95 |
| 85 | 79 | 92 |
| 90 | 77 | 88 |
| 95 | 70 | 74 |
| 100 | 53 | 64 |

**Table 8 specificities at important sensitivity thresholds in logistic regression models of the 5 most characteristic marker genes and of the 10 marker genes**

| **sensitivity thresholds(%)** | **specificity (%)** | |
|---|---|---|
| | **5 markers** | **10 markers** |
| 80 | 84 | 95 |
| 85 | 78 | 93 |
| 90 | 68 | 88 |
| 95 | 64 | 80 |
| 100 | 51 | 67 |

It should be noted that the detection results of the methylation levels provided in this Example were obtained with primer set 1 for each biomarker gene (for example, for APC gene, use APC primer set 1; for BRCA1 gene, use BRCA1 primer set 1, and so on), and similar detection results were obtained with other primer sets provided herein (data not shown).

The technical solutions provided by the present invention, through jointly detecting the methylation levels of one or more genes of APC, BRCA1, CCND2, CST6, GP5, GSTP1, PITX2, RARB, RASSF1A and SOX17 genes or fragments thereof, improved the sensitivity and specificity of breast cancer detection, and thus ensured the accuracy and reliability of the test results. Moreover, the detection of methylatation of above biomarker genes in a sample with the primers provided in the kit of the present invention was able to quickly and conveniently determine the sample was positive or not and the risk value by using a logistic regression equation analysis.

The above Examples are only used to illustrate the technical solutions of the present invention and not to limit them. It will be understood by those of ordinary skill in the art that the technical solutions described in the foregoing Examples can be modified, or some or all of the technical features can be replaced equivalently. These modifications or replacements do not deviate the essence of the corresponding technical solutions from the scope of the technical solutions of the Examples of the present invention, and they should all be encompassed within the scope of the present specification.

## Claims

1. A method for identifying a breast cancer status in a subject comprising the following steps:
1) collecting a biological sample from the subject;
2) detecting methylation level(s) of a biomarker gene in the biological sample, wherein the biomarker gene(s) is/are selected from one or more of the following genes: APC, BRCA1, CCND2, CST6, GP5, GSTP1, PITX2, RARB, RASSF1A and SOX17; and
3) comparing the methylation level(s) detected in step 2) with normal methylation level(s) of the corresponding biomarker gene(s) in a population to determine the breast cancer status in the subject.

2. The method of claim 1, further comprising performing steps 1) and 2) again after the subject undergoes a medical treatment, and comparing the both obtained detection results of the methylation level(s) to determine change of the breast cancer status in the subject.

3. The method of claim 1 or claim 2, wherein the breast cancer status includes breast cancer susceptibility and presence, progression, subtype, and/or stage of the breast cancer.

4. The method of claim 1 or claim 2, wherein step 2) comprises extracting DNA from the biological sample and treating the extracted DNA with a bisulfite, so that unmethylated cytosine residues in the DNA are deaminated, and methylated cytosine residues remain unchanged.

5. The method of claim 4, wherein the bisulfite is sodium bisulfite.

6. The method of claim 1 or claim 2, wherein in step 2) the biomarker genes are selected from 2 or more of APC, BRCA1, CCND2, CST6, GP5, GSTP1, PITX2, RARB, RASSF1A and SOX17.

7. The method of claim 6, wherein in step 2) the biomarker genes are selected from 5 or more of APC, BRCA1, CCND2, CST6, GP5, GSTP1, PITX2, RARB, RASSF1A and SOX17.

8. The method of claim 7, wherein in step 2) the biomarker genes are BRCA1, CCND2, PITX2, RARB and RASSF1A.

9. The method of claim 1 or claim 2, wherein the breast cancer status is breast cancer stage I or stage II, and the biomarker gene(s) is/are APC and/or RASSF1A.

10. The method of claim 1 or claim 2, wherein the breast cancer status is a noninfiltrating carcinoma, and the biomarker gene(s) is/are RARB and/or RASSF1A.

11. The method of claim 1 or claim 2, wherein the breast cancer status is an infiltrating ductal carcinoma, and the biomarker gene(s) is/are APC, CCND2 and/or RASSF1A.

12. The method of claim 1 or claim 2, wherein the breast cancer status is an infiltrating lobular carcinoma, and the biomarker gene(s) is/are CCND2 and/or RASSF1A.

13. The method of claim 1 or claim 2, wherein the breast cancer status is an infiltrating carcinoma other than infiltrating ductal carcinoma and infiltrating lobular carcinoma, and the biomarker gene(s) is/are CCND2 and/or RARB.

14. The method of claim 1 or claim 2, wherein step 2) comprises detecting the methylation level(s) of a target region within the biomarker gene(s), and wherein the target region is a nucleotide sequence of at least 15 bases in the biomarker gene(s), or a complementary sequence thereof.

15. The method of claim 1 or claim 2, wherein, in step 2),
the detection of the methylation level of the APC gene comprises the use of a primer pair having the sequences as set forth in SEQ ID NOs: 12 and 13 or a primer pair having the sequences as set forth in SEQ ID NOs: 16 and 17 to carry out a PCR amplification reaction, with the APC gene or a fragment thereof which is bisulfite-treated in the biological sample as a template;
the detection of the methylation level of the BRCA1 gene comprises the use of a primer pair having the sequences as set forth in SEQ ID NOs: 20 and 21, a primer pair having the sequences as set forth in SEQ ID NOs: 24 and 25 or a primer pair having the sequences as set forth in SEQ ID NOs: 28 and 29 to carry out a PCR amplification reaction, with the BRCA1 gene or a fragment thereof which is bisulfite-treated in the biological sample as a template;
the detection of the methylation level of the CCND2 gene comprises the use of a primer pair having the sequences as set forth in SEQ ID NOs: 32 and 33, a primer pair having the sequences as set forth in SEQ ID NOs: 36 and 37 or a primer pair having the sequences as set forth in SEQ ID NOs: 40 and 41 to carry out a PCR amplification reaction, with the CCND2 gene or a fragment thereof which is bisulfite-treated in the biological sample as a template;
the detection of the methylation level of the CST6 gene comprises the use of a primer pair having the sequences as set forth in SEQ ID NOs: 44 and 45 or a primer pair having the sequences as set forth in SEQ ID NOs: 48 and 49 to carry out a PCR amplification reaction, with the CST6 gene or a fragment thereof which is bisulfite-treated in the biological sample as a template;
the detection of the methylation level of the GP5 gene comprises the use of a primer pair having the sequences as set forth in SEQ ID NOs: 52 and 53, a primer pair having the sequences as set forth in SEQ ID NOs: 56 and 57 or a primer pair having the sequences as set forth in SEQ ID NOs: 60 and 61 to carry out a PCR amplification reaction, with the GP5 gene or a fragment thereof which is bisulfite-treated in the biological sample as a template;
the detection of the methylation level of the GSTP1 gene comprises the use of a primer pair having the sequences as set forth in SEQ ID NOs: 64 and 65 or a primer pair having the sequences as set forth in SEQ ID NOs: 68 and 69 to carry out a PCR amplification reaction, with the GSTP1 gene or a fragment thereof which is bisulfite-treated in the biological sample as a template;
the detection of the methylation level of the PITX2 gene comprises the use of a primer pair having the sequences as set forth in SEQ ID NOs: 72 and 73, a primer pair having the sequences as set forth in SEQ ID NOs: 76 and 77, a primer pair having the sequences as set forth in SEQ ID NOs: 80 and 81 or a primer pair having the sequences as set forth in SEQ ID NOs: 84 and 85 to carry out a PCR amplification reaction, with the PITX2 gene or a fragment thereof which is bisulfite-treated in the biological sample as a template;
the detection of the methylation level of the RARB gene comprises the use of a primer pair having the sequences as set forth in SEQ ID NOs: 88 and 89, a primer pair having the sequences as set forth in SEQ ID NOs: 92 and 93 or a primer pair having the sequences as set forth in SEQ ID NOs: 96 and 97 to carry out a PCR amplification reaction, with the RARB gene or a fragment thereof which is bisulfite-treated in the biological sample as a template;
the detection of the methylation level of the RASSF1A gene comprises the use of a primer pair having the sequences as set forth in SEQ ID NOs: 100 and 101, a primer pair having the sequences as set forth in SEQ ID NOs: 104 and 105 or a primer pair having the sequences as set forth in SEQ ID NOs: 108 and 109 to carry out a PCR amplification reaction, with the RASSF1A gene or a fragment thereof which is bisulfite-treated in the biological sample as a template; and
the detection of the methylation level of the SOX17 gene comprises the use of a primer pair having the sequences as set forth in SEQ ID NOs: 112 and 113, a primer pair having the sequences as set forth in SEQ ID NOs: 116 and 117 or a primer pair having the sequences as set forth in SEQ ID NOs: 120 and 121 to carry out a PCR amplification reaction, with the SOX17 gene or a fragment thereof which is bisulfite-treated in the biological sample as a template.

16. The method of claim 14, wherein, in the step 2),
the detection of the methylation level of the APC gene comprises the use of a primer pair having the sequences as set forth in SEQ ID NOs: 12 and 13 and a blocking primer having the sequence as set forth in SEQ ID NO: 14, or a primer pair having the sequences as set forth in SEQ ID NOs: 16 and 17 and a blocking primer having the sequence as set forth in SEQ ID NO: 18 to carry out a PCR amplification reaction, with the bisulfite-treated APC gene or a fragment thereof in the biological sample as a template;
the detection of the methylation level of the BRCA1 gene comprises the use of a primer pair having the sequences as set forth in SEQ ID NOs: 20 and 21 and a blocking primer having the sequence as set forth in SEQ ID NO: 22, a primer pair having the sequences as set forth in SEQ ID NOs: 24 and 25 and a blocking primer having the sequence as set forth in SEQ ID NO: 26 or a primer pair having the sequences as set forth in SEQ ID NOs: 28 and 29 and a blocking primer having the sequence as set forth in SEQ ID NO: 30 to carry out a PCR amplification reaction, with the bisulfite-treated BRCA1 gene or a fragment thereof in the biological sample as a template;
the detection of the methylation level of the CCND2 gene comprises the use of a primer pair having the sequences as set forth in SEQ ID NOs: 32 and 33 and a blocking primer having the sequence as set forth in SEQ ID NO: 34, a primer pair having the sequences as set forth in SEQ ID NOs: 36 and 37 and a blocking primer having the sequence as set forth in SEQ ID NO: 38 or a primer pair having the sequences as set forth in SEQ ID NOs: 40 and 41 and a blocking primer having the sequence as set forth in SEQ ID NO: 42 to carry out a PCR amplification reaction, with the bisulfite-treated CCND2 gene or a fragment thereof in the biological sample as a template;
the detection of the methylation level of the CST6 gene comprises the use of a primer pair having the sequences as set forth in SEQ ID NOs: 44 and 45 and a blocking primer having the sequence as set forth in SEQ ID NO: 46, or a primer pair having the sequences as set forth in SEQ ID NOs: 48 and 49 and a blocking primer having the sequence as set forth in SEQ ID NO: 50 to carry out a PCR amplification reaction, with the bisulfite-treated CST6 gene or a fragment thereof in the biological sample as a template;
the detection of the methylation level of the GP5 gene comprises the use of a primer pair having the sequences as set forth in SEQ ID NOs: 52 and 53 and a blocking primer having the sequence as set forth in SEQ ID NO: 54, a primer pair having the sequences as set forth in SEQ ID NOs: 56 and 57 and a blocking primer having the sequence as set forth in SEQ ID NO: 58 or a primer pair having the sequences as set forth in SEQ ID NOs: 60 and 61 and a blocking primer having the sequence as set forth in SEQ ID NO: 62 to carry out a PCR amplification reaction, with the bisulfite-treated GP5 gene or a fragment thereof in the biological sample as a template;
the detection of the methylation level of the GSTP1 gene comprises the use of a primer pair having the sequences as set forth in- SEQ ID NOs: 64 and 65 and a blocking primer having the sequence as set forth in SEQ ID NO: 66, or a primer pair having the sequences as set forth in SEQ ID NOs: 68 and 69 and a blocking primer having the sequence as set forth in SEQ ID NO: 70 to carry out a PCR amplification reaction, with the bisulfite-treated GSTP1 gene or a fragment thereof in the biological sample as a template;
the detection of the methylation level of the PITX2 gene comprises the use of a primer pair having the sequences as set forth in SEQ ID NOs: 72 and 73 and a blocking primer having the sequence as set forth in SEQ ID NO: 74, a primer pair having the sequences as set forth in SEQ ID NOs: 76 and 77 and a blocking primer having the sequence as set forth in SEQ ID NO: 78, a primer pair having the sequences as set forth in SEQ ID NOs: 80 and 81 and a blocking primer having the sequence as set forth in SEQ ID NO: 82 or a primer pair having the sequences as set forth in SEQ ID NOs: 84 and 85 and a blocking primer having the sequence as set forth in SEQ ID NO: 86 to carry out a PCR amplification reaction, with the bisulfite-treated PITX2 gene or a fragment thereof in the biological sample as a template;
the detection of the methylation level of the RARB gene comprises the use of a primer pair having the sequences as set forth in SEQ ID NOs: 88 and 89 and a blocking primer having the sequence as set forth in SEQ ID NO: 90, a primer pair having the sequences as set forth in SEQ ID NOs: 92 and 93 and a blocking primer having the sequence as set forth in SEQ ID NO: 94, or a primer pair having the sequences as set forth in SEQ ID NOs: 96 and 97 and a blocking primer having the sequence as set forth in SEQ ID NO: 98 to carry out a PCR amplification reaction, with the bisulfite-treated RARB gene or a fragment thereof in the biological sample as a template;
the detection of the methylation level of the RASSF1A gene comprises the use of a primer pair having the sequences as set forth in SEQ ID NOs: 100 and 101 and a blocking primer having the sequence as set forth in SEQ ID NO: 102, or a primer pair having the sequences as set forth in SEQ ID NOs: 104 and 105 and a blocking primer having the sequence as set forth in SEQ ID NO: 106 to carry out a PCR amplification reaction, with the bisulfite-treated RASSF1A gene or a fragment thereof in the biological sample as a template; and
the detection of the methylation level of the SOX17 gene comprises the use of a primer pair having the sequences as set forth in SEQ ID NOs: 112 and 113 and a blocking primer having the sequence as set forth in SEQ ID NO: 114, a primer pair having the sequences as set forth in SEQ ID NOs: 116 and 117 and a blocking primer having the sequence as set forth in SEQ ID NO: 118 or a primer pair having the sequences as set forth in SEQ ID NOs: 120 and 121 and a blocking primer having the sequence as set forth in SEQ ID NO: 122 to carry out a PCR amplification reaction, with the bisulfite-treated SOX17 gene or a fragment thereof in the biological sample as a template,
wherein the blocking primers have a 3' end modification which prevents the extension and amplification of a DNA polymerase.

17. The method of claim 16, wherein, in the step 2),
the detection of the methylation level of the APC gene comprises the use of a primer pair having the sequences as set forth in SEQ ID NOs: 12 and 13, a blocking primer having the sequence as set forth in SEQ ID NO: 14 and a probe having the sequence as set forth in SEQ ID NO: 15; or a primer pair having the sequences as set forth in SEQ ID NOs: 16 and 17, a blocking primer having the sequence as set forth in SEQ ID NO: 18 and a probe having the sequence as set forth in SEQ ID NO: 19 to carry out a PCR amplification reaction, with the bisulfite-treated APC gene or a fragment thereof in the biological sample as a template;
the detection of the methylation level of the BRCA1 gene comprises the use of a primer pair having the sequences as set forth in SEQ ID NOs: 20 and 21, a blocking primer having the sequence as set forth in SEQ ID NO: 22 and a probe having the sequence as set forth in SEQ ID NO: 23; a primer pair having the sequences as set forth in SEQ ID NOs: 24 and 25, a blocking primer having the sequence as set forth in SEQ ID NO: 26 and a probe having the sequence as set forth in SEQ ID NO: 27; or a primer pair having the sequences as set forth in SEQ ID NOs: 28 and 29, a blocking primer having the sequence as set forth in SEQ ID NO: 30 and a probe having the sequence as set forth in SEQ ID NO: 31 to carry out a PCR amplification reaction, with the bisulfite-treated BRCA1 gene or a fragment thereof in the biological sample as a template;
the detection of the methylation level of the CCND2 gene comprises the use of a primer pair having the sequences as set forth in SEQ ID NOs: 32 and 33, a blocking primer having the sequence as set forth in SEQ ID NO: 34 and a probe having the sequence as set forth in SEQ ID NO: 35; a primer pair having the sequences as set forth in SEQ ID NOs: 36 and 37, a blocking primer having the sequence as set forth in SEQ ID NO: 38 and a probe having the sequence as set forth in SEQ ID NO: 39; or a primer pair having the sequences as set forth in SEQ ID NOs: 40 and 41, a blocking primer having the sequence as set forth in SEQ ID NO: 42 and a probe having the sequence as set forth in SEQ ID NO: 43 to carry out a PCR amplification reaction, with the bisulfite-treated CCND2 gene or a fragment thereof in the biological sample as a template;
the detection of the methylation level of the CST6 gene comprises the use of a primer pair having the sequences as set forth in SEQ ID NOs: 44 and 45, a blocking primer having the sequence as set forth in SEQ ID NO: 46 and a probe having the sequence as set forth in SEQ ID NO: 47; or a primer pair having the sequences as set forth in SEQ ID NOs: 48 and 49, a blocking primer having the sequence as set forth in SEQ ID NO: 50 and a probe having the sequence as set forth in SEQ ID NO: 51 to carry out a PCR amplification reaction, with the bisulfite-treated CST6 gene or a fragment thereof in the biological sample as a template;
the detection of the methylation level of the GP5 gene comprises the use of a primer pair having the sequences as set forth in SEQ ID NOs: 52 and 53, a blocking primer having the sequence as set forth in SEQ ID NO: 54 and a probe having the sequence as set forth in SEQ ID NO: 55; a primer pair having the sequences as set forth in SEQ ID NOs: 56 and 57, a blocking primer having the sequence as set forth in SEQ ID NO: 58 and a probe having the sequence as set forth in SEQ ID NO: 59; or a primer pair having the sequences as set forth in SEQ ID NOs: 60 and 61, a blocking primer having the sequence as set forth in SEQ ID NO: 62 and a probe having the sequence as set forth in SEQ ID NO: 63 to carry out a PCR amplification reaction, with the bisulfite-treated GP5 gene or a fragment thereof in the biological sample as a template;
the detection of the methylation level of the GSTP1 gene comprises the use of a primer pair having the sequences as set forth in SEQ ID NOs: 64 and 65, a blocking primer having the sequence as set forth in SEQ ID NO: 66 and a probe having the sequence as set forth in SEQ ID NO: 67; or a primer pair having the sequences as set forth in SEQ ID NOs: 68 and 69, a blocking primer having the sequence as set forth in SEQ ID NO: 70 and a probe having the sequence as set forth in SEQ ID NO: 71 to carry out a PCR amplification reaction, with the bisulfite-treated GSTP1 gene or a fragment thereof in the biological sample as a template;
the detection of the methylation level of the PITX2 gene comprises the use of a primer pair having the sequences as set forth in SEQ ID NOs: 72 and 73, a blocking primer having the sequence as set forth in SEQ ID NO: 74 and a probe having the sequence as set forth in SEQ ID NO: 75; a primer pair having the sequences as set forth in SEQ ID NOs: 76 and 77, a blocking primer having the sequence as set forth in SEQ ID NO: 78 and a probe having the sequence as set forth in SEQ ID NO: 79; a primer pair having the sequences as set forth in SEQ ID NOs: 80 and 81, a blocking primer having the sequence as set forth in SEQ ID NO: 82 and a probe having the sequence as set forth in SEQ ID NO: 83; or a primer pair having the sequences as set forth in SEQ ID NOs: 84 and 85, a blocking primer having the sequence as set forth in SEQ ID NO: 86 and a probe having the sequence as set forth in SEQ ID NO: 87to carry out a PCR amplification reaction, with the bisulfite-treated PITX2 gene or a fragment thereof in the biological sample as a template;
the detection of the methylation level of the RARB gene comprises the use of a primer pair having the sequences as set forth in SEQ ID NOs: 88 and 89, a blocking primer having the sequence as set forth in SEQ ID NO: 90 and a probe having the sequence as set forth in SEQ ID NO: 91; a primer pair having the sequences as set forth in SEQ ID NOs: 92 and 93, a blocking primer having the sequence as set forth in SEQ ID NO: 94 and a probe having the sequence as set forth in SEQ ID NO: 95; or a primer pair having the sequences as set forth in SEQ ID NOs: 96 and 97, a blocking primer having the sequence as set forth in SEQ ID NO: 98 and a probe having the sequence as set forth in SEQ ID NO: 99 to carry out a PCR amplification reaction, with the bisulfite-treated RARB gene or a fragment thereof in the biological sample as a template;
the detection of the methylation level of the RASSF1A gene comprises the use of a primer pair having the sequences as set forth in SEQ ID NOs: 100 and 101, a blocking primer having the sequence as set forth in SEQ ID NO: 102 and a probe having the sequence as set forth in SEQ ID NO: 103; a primer pair having the sequences as set forth in SEQ ID NOs: 104 and 105, a blocking primer having the sequence as set forth in SEQ ID NO: 106 and a probe having the sequence as set forth in SEQ ID NO: 107; or a primer pair having the sequences as set forth in SEQ ID NOs: 108 and 109, a blocking primer having the sequence as set forth in SEQ ID NO: 110 and a probe having the sequence as set forth in SEQ ID NO: 111 to carry out a PCR amplification reaction, with the bisulfite-treated RASSF1A gene or a fragment thereof in the biological sample as a template; and
the detection of the methylation level of the SOX17 gene comprises the use of a primer pair having the sequences as set forth in SEQ ID NOs: 112 and 113, a blocking primer having the sequence as set forth in SEQ ID NO: 114 and a probe having the sequence as set forth in SEQ ID NO: 115; a primer pair having the sequences as set forth in SEQ ID NOs: 116 and 117, a blocking primer having the sequence as set forth in SEQ ID NO: 118 and a probe having the sequence as set forth in SEQ ID NO: 119; or a primer pair having the sequences as set forth in SEQ ID NOs: 120 and 121, a blocking primer having the sequence as set forth in SEQ ID NO: 122 and a probe having the sequence as set forth in SEQ ID NO: 123 to carry out a PCR amplification reaction, with the bisulfite-treated SOX17 gene or a fragment thereof in the biological sample as a template,
wherein the probes have a fluorescent group at one end and a fluorescence quenching group at the other end.

18. The method of claim 1 or claim 2, wherein the step 2) further comprises the use of a primer pair having the sequences as set forth in SEQ ID NOs: 124 and 125 and a probe having the sequence as set forth in SEQ ID NO: 126 to carry out a PCR amplification reaction, with a bisulfite-treated ACTB gene or a fragment thereof used as an internal reference gene in the biological sample as a template.

19. The method of claim 1 or claim 2, wherein the step 3) comprises determining the breast cancer status in the subject according to the methylation levels of the biomarker genes based on a logistic regression.

20. The method of claim 1 or claim 2, wherein the biological sample is selected from blood, serum, plasma, breast duct fluid, lymph, cerebrospinal fluid, urine, and tissue biopsy from the subject.

21. A kit for identifying a breast cancer status in a subject comprising a primer pair for detecting methylation level(s) of a biomarker gene in a biological sample from the subject, wherein the primer pair is used to carry out a PCR amplification reaction with the biomarker gene or a fragment thereof which is bisulfite-treated as a template; and the biomarker gene(s) is/are selected from one or more of the following genes: APC, BRCA1, CCND2, CST6, GP5, GSTP1, PITX2, RARB, RASSF1A and SOX17.

22. The kit of claim 21, wherein the biomarker genes are selected from 2 or more of APC, BRCA1, CCND2, CST6, GP5, GSTP1, PITX2, RARB, RASSF1A and SOX17.

23. The kit of claim 21, wherein the biomarker genes are selected from 5 or more of APC, BRCA1, CCND2, CST6, GP5, GSTP1, PITX2, RARB, RASSF1A and SOX17.

24. The kit of claim 22, wherein the biomarker genes are BRCA1, CCND2, PITX2, RARB and RASSF1A.

25. The kit of claim 21, wherein the breast cancer status is breast cancer stage I or stage II, and the biomarker gene(s) is/are APC and/or RASSF1A.

26. The kit of claim 20, wherein the breast cancer status is a noninfiltrating carcinoma, and the biomarker gene(s) is/are RARB and/or RASSF1A.

27. The kit of claim 21, wherein the breast cancer status is an infiltrating ductal carcinoma, and the biomarker gene(s) is/are APC, CCND2 and/or RASSF1A.

28. The kit of claim 21, wherein the breast cancer status is an infiltrating lobular carcinoma, and the biomarker gene(s) is/are CCND2 and/or RASSF1A.

29. The kit of claim 21, wherein the breast cancer status is an infiltrating carcinoma other than infiltrating ductal carcinoma and infiltrating lobular carcinoma, and the biomarker gene(s) is/are CCND2 and/or RARB.

30. The kit of claim 21, wherein
the primer pair used for the the detection of the methylation level of APC has the sequences as set forth in SEQ ID NOs: 12 and 13 or the sequences as set forth in SEQ ID NOs: 16 and 17;
the primer pair used for the the detection of the methylation level of BRCA1 has the sequences as set forth in SEQ ID NOs: 20 and 21, the sequences as set forth in SEQ ID NOs: 24 and 25 or the sequences as set forth in SEQ ID NOs: 28 and 29;
the primer pair used for the the detection of the methylation level of CCND2 has the sequences as set forth in SEQ ID NOs: 32 and 33, the sequences as set forth in SEQ ID NOs: 36 and 37 or the sequences as set forth in SEQ ID NOs: 40 and 41;
the primer pair used for the the detection of the methylation level of CST6 has the sequences as set forth in SEQ ID NOs: 44 and 45 or the sequences as set forth in SEQ ID NOs: 48 and 49;
the primer pair used for the the detection of the methylation level of GP5 has the sequences as set forth in SEQ ID NOs: 52 and 53, the sequences as set forth in SEQ ID NOs: 56 and 57 or has the sequences as set forth in SEQ ID NOs: 60 and 61;
the primer pair used for the the detection of the methylation level of GSTP1 has the sequences as set forth in SEQ ID NOs: 64 and 65 or the sequences as set forth in SEQ ID NOs: 68 and 69;
the primer pair used for the the detection of the methylation level of PITX2 has the sequences as set forth in SEQ ID NOs: 72 and 73, the sequences as set forth in SEQ ID NOs: 76 and 77, the sequences as set forth in SEQ ID NOs: 80 and 81 or the sequences as set forth in SEQ ID NOs: 84 and 85;
the primer pair used for the the detection of the methylation level of RARB has the sequences as set forth in SEQ ID NOs: 88 and 89, the sequences as set forth in SEQ ID NOs: 92 and 93 or the sequences as set forth in SEQ ID NOs: 96 and 97;
the primer pair used for the the detection of the methylation level of RASSF1A has the sequences as set forth in SEQ ID NOs: 100 and 101, the sequences as set forth in SEQ ID NOs: 104 and 105 or the sequences as set forth in SEQ ID NOs: 108 and 109; and
the primer pair used for the the detection of the methylation level of SOX17 has the sequences as set forth in SEQ ID NOs: 112 and 113, the sequences as set forth in SEQ ID NOs: 116 and 117 or the sequences as set forth in SEQ ID NOs: 120 and 121.

31. The kit of claim 30 further comprising a blocking primer, wherein
the blocking primer used in combination with the primer pair having the sequences as set forth in SEQ ID NOs: 12 and 13 has the sequence as set forth in SEQ ID NO: 14;
the blocking primer used in combination with the primer pair having the sequences as set forth in SEQ ID NOs: 16 and 17 has the sequence as set forth in SEQ ID NO: 18;
the blocking primer used in combination with the primer pair having the sequences as set forth in SEQ ID NOs: 20 and 21 has the sequence as set forth in SEQ ID NO: 22;
the blocking primer used in combination with the primer pair having the sequences as set forth in SEQ ID NOs: 24 and 25 has the sequence as set forth in SEQ ID NO: 26;
the blocking primer used in combination with the primer pair having the sequences as set forth in SEQ ID NOs: 28 and 29 has the sequence as set forth in SEQ ID NO: 30;
the blocking primer used in combination with the primer pair having the sequences as set forth in SEQ ID NOs: 32 and 33 has the sequence as set forth in SEQ ID NO: 34;
the blocking primer used in combination with the primer pair having the sequences as set forth in SEQ ID NOs: 36 and 37 has the sequence as set forth in SEQ ID NO: 38;
the blocking primer used in combination with the primer pair having the sequences as set forth in SEQ ID NOs: 40 and 41 has the sequence as set forth in SEQ ID NO: 42;
the blocking primer used in combination with the primer pair having the sequences as set forth in SEQ ID NOs: 44 and 45 has the sequence as set forth in SEQ ID NO: 46;
the blocking primer used in combination with the primer pair having the sequences as set forth in SEQ ID NOs: 48 and 49 has the sequence as set forth in SEQ ID NO: 50;
the blocking primer used in combination with the primer pair having the sequences as set forth in SEQ ID NOs: 52 and 53 has the sequence as set forth in SEQ ID NO: 54;
the blocking primer used in combination with the primer pair having the sequences as set forth in SEQ ID NOs: 56 and 57 has the sequence as set forth in SEQ ID NO: 58;
the blocking primer used in combination with the primer pair having the sequences as set forth in SEQ ID NOs: 60 and 61 has the sequence as set forth in SEQ ID NO: 62;
the blocking primer used in combination with the primer pair having the sequences as set forth in SEQ ID NOs: 64 and 65 has the sequence as set forth in SEQ ID NO: 66;
the blocking primer used in combination with the primer pair having the sequences as set forth in SEQ ID NOs: 68 and 69 has the sequence as set forth in SEQ ID NO: 70;
the blocking primer used in combination with the primer pair having the sequences as set forth in SEQ ID NOs: 72 and 73 has the sequence as set forth in SEQ ID NO: 74;
the blocking primer used in combination with the primer pair having the sequences as set forth in SEQ ID NOs: 76 and 77 has the sequence as set forth in SEQ ID NO: 78;
the blocking primer used in combination with the primer pair having the sequences as set forth in SEQ ID NOs: 80 and 81 has the sequence as set forth in SEQ ID NO: 82;
the blocking primer used in combination with the primer pair having the sequences as set forth in SEQ ID NOs: 84 and 85 has the sequence as set forth in SEQ ID NO: 86;
the blocking primer used in combination with the primer pair having the sequences as set forth in SEQ ID NOs: 88 and 89 has the sequence as set forth in SEQ ID NO: 90;
the blocking primer used in combination with the primer pair having the sequences as set forth in SEQ ID NOs: 92 and 93 has the sequence as set forth in SEQ ID NO: 94;
the blocking primer used in combination with the primer pair having the sequences as set forth in SEQ ID NOs: 96 and 97 has the sequence as set forth in SEQ ID NO: 98;
the blocking primer used in combination with the primer pair having the sequences as set forth in SEQ ID NOs: 100 and 101 has the sequence as set forth in SEQ ID NO: 102;
the blocking primer used in combination with the primer pair having the sequences as set forth in SEQ ID NOs: 104 and 105 has the sequence as set forth in SEQ ID NO: 106;
the blocking primer used in combination with the primer pair having the sequences as set forth in SEQ ID NOs: 108 and 109 has the sequence as set forth in SEQ ID NO: 110;
the blocking primer used in combination with the primer pair having the sequences as set forth in SEQ ID NOs: 112 and 113 has the sequence as set forth in SEQ ID NO: 114;
the blocking primer used in combination with the primer pair having the sequences as set forth in SEQ ID NOs: 116 and 117 has the sequence as set forth in SEQ ID NO: 118; and
the blocking primer used in combination with the primer pair having the sequences as set forth in SEQ ID NOs: 120 and 121 has the sequence as set forth in SEQ ID NO: 122;
wherein the blocking primers have a 3' end modification which prevents the extension and amplification of a DNA polymerase.

32. The kit of claim 30 or claim 31 further comprising a probe, wherein
the probe used in combination with the primer pair having the sequences as set forth in SEQ ID NOs: 12 and 13 has the sequence as set forth in SEQ ID NO: 15;
the probe used in combination with the primer pair having the sequences as set forth in SEQ ID NOs: 16 and 17 has the sequence as set forth in SEQ ID NO: 19;
the probe used in combination with the primer pair having the sequences as set forth in SEQ ID NOs: 20 and 21 has the sequence as set forth in SEQ ID NO: 23;
the probe used in combination with the primer pair having the sequences as set forth in SEQ ID NOs: 24 and 25 has the sequence as set forth in SEQ ID NO: 27;
the probe used in combination with the primer pair having the sequences as set forth in SEQ ID NOs: 28 and 29 has the sequence as set forth in SEQ ID NO: 31;
the probe used in combination with the primer pair having the sequences as set forth in SEQ ID NOs: 32 and 33 has the sequence as set forth in SEQ ID NO: 35;
the probe used in combination with the primer pair having the sequences as set forth in SEQ ID NOs: 36 and 37 has the sequence as set forth in SEQ ID NO: 39;
the probe used in combination with the primer pair having the sequences as set forth in SEQ ID NOs: 40 and 41 has the sequence as set forth in SEQ ID NO: 43;
the probe used in combination with the primer pair having the sequences as set forth in SEQ ID NOs: 44 and 45 has the sequence as set forth in SEQ ID NO: 47;
the probe used in combination with the primer pair having the sequences as set forth in SEQ ID NOs: 48 and 49 has the sequence as set forth in SEQ ID NO: 51;
the probe used in combination with the primer pair having the sequences as set forth in SEQ ID NOs: 52 and 53 has the sequence as set forth in SEQ ID NO: 55;
the probe used in combination with the primer pair having the sequences as set forth in SEQ ID NOs: 56 and 57 has the sequence as set forth in SEQ ID NO: 59;
the probe used in combination with the primer pair having the sequences as set forth in SEQ ID NOs: 60 and 61 has the sequence as set forth in SEQ ID NO: 63;
the probe used in combination with the primer pair having the sequences as set forth in SEQ ID NOs: 64 and 65 has the sequence as set forth in SEQ ID NO: 67;
the probe used in combination with the primer pair having the sequences as set forth in SEQ ID NOs: 68 and 69 has the sequence as set forth in SEQ ID NO: 71;
the probe used in combination with the primer pair having the sequences as set forth in SEQ ID NOs: 72 and 73 has the sequence as set forth in SEQ ID NO: 75;
the probe used in combination with the primer pair having the sequences as set forth in SEQ ID NOs: 76 and 77 has the sequence as set forth in SEQ ID NO: 79;
the probe used in combination with the primer pair having the sequences as set forth in SEQ ID NOs: 80 and 81 has the sequence as set forth in SEQ ID NO: 83;
the probe used in combination with the primer pair having the sequences as set forth in SEQ ID NOs: 84 and 85 has the sequence as set forth in SEQ ID NO: 87;
the probe used in combination with the primer pair having the sequences as set forth in SEQ ID NOs: 88 and 89 has the sequence as set forth in SEQ ID NO: 91;
the probe used in combination with the primer pair having the sequences as set forth in SEQ ID NOs: 92 and 93 has the sequence as set forth in SEQ ID NO: 95;
the probe used in combination with the primer pair having the sequences as set forth in SEQ ID NOs: 96 and 97 has the sequence as set forth in SEQ ID NO: 99;
the probe used in combination with the primer pair having the sequences as set forth in SEQ ID NOs: 100 and 101 has the sequence as set forth in SEQ ID NO: 103;
the probe used in combination with the primer pair having the sequences as set forth in SEQ ID NOs: 104 and 105 has the sequence as set forth in SEQ ID NO: 107;
the probe used in combination with the primer pair having the sequences as set forth in SEQ ID NOs: 108 and 109 has the sequence as set forth in SEQ ID NO: 111;
the probe used in combination with the primer pair having the sequences as set forth in SEQ ID NOs: 112 and 113 has the sequence as set forth in SEQ ID NO: 115;
the probe used in combination with the primer pair having the sequences as set forth in SEQ ID NOs: 116 and 117 has the sequence as set forth in SEQ ID NO: 119; and
the probe used in combination with the primer pair having the sequences as set forth in SEQ ID NOs: 120 and 121 has the sequence as set forth in SEQ ID NO: 123,
wherein the probes have a fluorescent group at one end and a fluorescence quenching group at the other end.

33. The kit of claim 21, further comprising a primer pair having the sequences as set forth in SEQ ID NOs: 124 and 125 and a probe having the sequence as set forth in SEQ ID NO: 126, for carrying out a PCR amplification reaction with a bisulfite-treated ACTB gene or a fragment thereof used as an internal reference gene in the biological sample as a template.

34. The kit of claim 21, further comprising a DNA extraction reagent and a bisulfite reagent.

35. The kit of claim 34, wherein the bisulfite reagent comprises sodium bisulfite.

36. The kit of claim 21, wherein the breast cancer status includes breast cancer susceptibility and presence, progression, subtype, and/or stage of the breast cancer.

37. The kit of claim 21, wherein the biological sample is selected from blood, serum, plasma, breast duct fluid, lymph, cerebrospinal fluid, urine, and tissue biopsy from the subject.

38. The kit of claim 21, further comprising an instruction that describes how to use the kit and process detection results with a logistic regression.


SEQUENCE LISTING

<110>  BEIJING EXELLON MEDICAL TECHNOLOGY CO., LTD

<120>  METHOD AND KIT FOR IDENTIFYING BREAST CANCER STATUS

<130>  20603US

<160>  126

<170>  PatentIn version 3.5

<210>  1
<211>  483
<212>  DNA
<213>  Homo sapiens

<400>  1
gagatgtaat ttattactct ccctcccacc tccggcatct tgtgctaatc cttctgccct      60

gcggacctcc cccgactctt tactatgcgt gtcaactgcc atcaacttcc ttgcttgctg     120

gggactgggg ccgcgagggc atacccccga ggggtacggg gctagggcta ggcaggctgt     180

gcggttgggc ggggccctgt gccccactgc ggagtgcggg tcgggaagcg gagagagaag     240

cagctgtgta atccgctgga tgcggaccag ggcgctcccc attcccgtcg ggagcccgcc     300

gattggctgg gtgtgggcgc acgtgaccga catgtggctg tattggtgca gcccgccagg     360

gtgtcactgg agacagaatg gaggtgctgc cggactcgga aatggggtag gtgctggagc     420

caccatggcc aggcttgctg cggggggagg ggggaaggtg gttttccctc gcactgtctt     480

aaa                                                                   483

<210>  2
<211>  828
<212>  DNA
<213>  Homo sapiens

<400>  2
cctcttccgt ctctttcctt ttacgtcatc cgggggcaga ctgggtggcc aatccagagc      60

cccgagagac gcttggctct ttctgtccct cccatcctct gattgtacct tgatttcgta     120

ttctgagagg ctgctgctta gcggtagccc cttggtttcc gtggcaacgg aaaagcgcgg     180

gaattacaga taaattaaaa ctgcgactgc gcggcgtgag ctcgctgaga cttcctggac     240

gggggacagg ctgtggggtt tctcagataa ctgggcccct gcgctcagga ggccttcacc     300

ctctgctctg ggtaaaggta gtagagtccc gggaaaggga cagggggccc aagtgatgct     360

ctggggtact ggcgtgggag agtggatttc cgaagctgac agatgggtat tctttgacgg     420

ggggtagggg cggaacctga gaggcgtaag gcgttgtgaa ccctggggag gggggcagtt     480

tgtaggtcgc gagggaagcg ctgaggatca ggaagggggc actgagtgtc cgtgggggaa     540

tcctcgtgat aggaactgga atatgccttg agggggacac tatgtcttta aaaacgtcgg     600

ctggtcatga ggtcaggagt tccagaccag cctgaccaac gtggtgaaac tccgtctcta 660

ctaaaaatac aaaaattagc cgggcgtggt gccgctccag ctactcagga ggctgaggca 720

ggagaatcgc tagaacccgg gaggcggagg ttgcagtgag ccgagatcgc gccattgcac 780

tccagcctgg gcgacagagc gagactgtct caaaacaaaa caaaacaa 828

<210> 3
<211> 897
<212> DNA
<213> Homo sapiens

<400> 3
aagaggctgg ctccggggcc tgagttaatc gcttgcacct ctagtttatt cgctcccctc 60

ctccgccttg cagggaacct agtgtacggc tcacccagcc cgcgccccac cccgccttgc 120

tggctctccg cgcccctgcc cgggccccct ctctcggtga gggaggcact cagtcggcct 180

cggtgtgccc agagagctcg agccacgcca tgcccgctgc acgtgccagc ttggccagca 240

catcagggcg ctggtctctc cccttcctcc tggagtgaaa tacaccaaag ggcgcggtgg 300

gggtgggggg tgacggaggg aaggaggtga agaaacgcca ccagatcgta tctcctgtaa 360

agacagcctt gactcaagca tgcgttagag cacgtgtcag ggccgaccgt gctggcggcg 420

acttcaccgc agtcggctcc cagggagaaa gcctggcgag tgaggcgcga aaccggaggg 480

gtcggcgagg atgcgggcga aggaccgagc gtggaggcct catgcctccg gggaaaggaa 540

ggggtggtgg tgtttgcgca gggggagcga gggggagccg gacctaatcc ctcactcgcc 600

ccctccccct cccgggccat ttcctagaaa gctgcatcgg tgtggccacg ctcagcgcag 660

acacctcggg cggcttgtca gcagatgcag gggcgaggaa gcgggttttt cctgcgtggc 720

cgctggccgc gggggaaccg ctgggagccc tgcccccggc ctgcggcggc cctagacgct 780

gcaccgcgtc gccccacggc gcccgaagag cccccagaaa cacgatggtt tctgctcgag 840

gatcacattc tatccctcca gagaagcacc ccccttcctt cctaataccc acctctc 897

<210> 4
<211> 621
<212> DNA
<213> Homo sapiens

<400> 4
gtaagagccc acgaagagct gcggttggta gttcattctg gacagccctc ccgtgaaccg 60

tccctgtact ggcacttgtt gctggggact gtcgctgtcc tctccctccc cgggccaggt 120

gtgtcctgga gggcagggaa gcgtcttggc acgcgggtgc gcgccgcccc ctcggcctcc 180

tgggctccct gaacctcgca ggaccccggc aacttcgagc cccgccccag ctccaggccg 240

cgggggcgca tcgcgggcgt cgggcggggc ggcccagcgg gtaaaagctg cgcggccgca 300

agctcggcac tcacggctct gagggctccg acggcactga cggccatggc gcgttcgaac 360

ctcccgctgg cgctgggcct ggccctggtc gcattctgcc tcctggcgct gccacgcgac 420

gcccgggccc ggccgcagga gcgcatggtc ggagaactcc gggacctgtc gcccgacgac 480

ccgcaggtgc agaaggcggc gcaggcggcc gtggccagct acaacatggg cagcaacagc 540

atctactact tccgagacac gcacatcatc aaggcgcaga gccaggtgcg gcgggcgggg 600

tgctgggagg ggacacccgg c 621

<210> 5
<211> 1050
<212> DNA
<213> Homo sapiens

<400> 5
ccgcgggctc agagtcaccc ctaagtaccg caggcggctc aggttgcgga aggcggcggc 60

gggcagggtg cgcagctggg tgcggttcag ccacagctcc tgcaggcccc ccatctcccc 120

gaagagcacc cccgggagct ctgccagcgg gttctcgaac agagtcaaca gagtcagatt 180

gtgcgaatga agaaagagcg cagaggggag aaacgcaagg tggtttctcg aaagcgtcaa 240

agaactgagg tttgggagcc ggtcgaaggc cccgggtgcg atggaacgga tgtgatttcg 300

gtggaactgc agctccgtca gggcgcccag gctgttcaac agccccgaat ccagagacac 360

aaggcggttc gagtggagca gaagtctctc gagcttagcc tgtgctccaa gcaacccctt 420

gggcaggtgg gtcaggttgt ttcccgataa atccaacaac ttcaggttct ccagattcgt 480

gaagagactg gcaggaagga aatcgagctg attctggttc agagcgagct cctgcaggtt 540

aaccagtttc tgaaacatgt tttggtcaat gccccttagc gcattgtggt ccaaaaacaa 600

ctgctccagg agcaccatct tatccagcag cgcacctgga agatgcgtga ttttgttgcg 660

cgacagcctc agggttttca gttttatcag gtcactgaag gtgccggggg caacggcgga 720

aatgtggctg tcggagatca tgaggcgctg caggacggtc atgccgctga agctctggct 780

ctgcaggacg ccgcggccca ttccgaagag caggatgtgc gtgaggttgg tgggcaggcc 840

tagcgcggag atgcgcgcca cgtcgccccc cgagcactgc gcggcgtccc ggaagacaca 900

cttgcaagct ggcggacagg ggaagggctg ggcgcgcaga agcccgagca ccgcgcacag 960

tagagtcccc ctcagcatgt ctgaaaaagc aaccgtggga gtgtggtcaa cacacaggag 1020

cgttcgcgcc tgtactgaac cctgggatcc 1050

<210> 6
<211> 730
<212> DNA
<213> Homo sapiens

<400> 6
tgaagcgggt gtgcaagctc cgggatcgca gcggtcttag ggaatttccc cccgcgatgt 60

cccggcgcgc cagttcgctg cgcacacttc gctgcggtcc tcttcctgct gtctgtttac 120

tccctaggcc ccgctgggga cctgggaaag agggaaaggc ttccccggcc agctgcgcgg 180

cgactccggg gactccaggg cgcccctctg cggccgacgc ccggggtgca gcggccgccg 240

gggctggggc cggcgggagt ccgcgggacc ctccagaaga gcggccggcg ccgtgactca 300

gcactggggc ggagcggggc gggaccaccc ttataaggct cggaggccgc gaggccttcg 360

ctggagtttc gccgccgcag tcttcgccac cagtgagtac gcgcggcccg cgtccccggg 420

gatggggctc agagctccca gcatggggcc aacccgcagc atcaggcccg ggctcccggc 480

agggctcctc gcccacctcg agacccggga cgggggccta ggggacccag gacgtcccca 540

gtgccgttag cggctttcag gggcccggga gcgcctcggg gagggatggg accccggggg 600

cggggagggg gggcagactg cgctcaccgc gccttggcat cctcccccgg gctccagcaa 660

acttttcttt gttcgctgca gtgccgccct acaccgtggt ctatttccca gttcgaggta 720

ggagcatgtg 730

<210> 7
<211> 550
<212> DNA
<213> Homo sapiens

<400> 7
actccgtgtg gggagtgacg tgacgtcagc agagattcca ccaaactcca ctgcacagtg 60

gcgcgcgggc ggccggccga gcccggctgc gcggctggcg atccaggagc gagcacagcg 120

cccgggcgag cgccgggggg agcgagcagg ggcgacgaga aacgaggcag gggagggaag 180

cagatgccag cgggccgaag agtcgggagc cggagccggg agagcgaaag gagaggggac 240

ctggcggggc acttaggagc caaccgagga gcaggagcac ggactcccac tgtggaaagg 300

aggaccagaa gggaggatgg gatggaagag aagaaaaagc aatctgcgcc aacccggcag 360

ccctaataaa tcaaaggggg agcgccaggg cagcggggag acagaaacgt acttttgggg 420

agcaaatcag gacgggctgg gaggaagcga cagggaaagt ggcccaagag acggaacaaa 480

ggacaatgtt catggggttg tttgggacga ggcgtgtgga gtgtgggtgt gagcgtgcgt 540

gtgtgacctt 550

<210> 8
<211> 552
<212> DNA
<213> Homo sapiens

<400> 8
   tggaggagtc cagcgaccgg ggctgaccgg gagccagaac cgaagccatg gctaacggct 60
   ggggatggtg acaggaagat gaggagacgg ccgacagctt ggtccccgct gctcggtgct 120
   ccaagtgaag cgggcctttc atgcagttca tggacgaggg agcgcgacgc tctactagtc 180
   cttggctact gccccgccga gcccccgtag ccgccgctgc ccgctccggg tcgcgctcta 240
   ggcgcggagt ttccccgctg cggggagagc caggggacgc aacccccgcc gagttctcaa 300
   gccaagctgc ccccgtctcc tccggaaggc tcaagcgaaa aagtccggag acggaaagtc 360
   agcgggcaaa cgaagacatg ggatgtgggc agaagggcac cactcagagc gtctttaggg 420
   agcaggcttc caagctccaa agcgaaacaa gagtgggcaa agaccccctt cttctctccc 480
   tccctccccc aagaacccct ccaataagga aagctaacgc cgaccgcgct ctgcccgccc 540
   cccccccacg cg 552

<210> 9
<211> 828
<212> DNA
<213> Homo sapiens

<400> 9
acagacagaa aggcgcacag aggaatttaa agtgtgggct ggggggcgag gcggtgggcg 60

ggaggcgagc gggcgcaggc ggaacaccgt tttccaagct aagccgccgc aaataaaaag 120

gcgtaaaggg agagaagttg gtgctcaacg tgagccagga gcagcgtccc ggctcctccc 180

ctgctcattt taaaagcact tcttgtattg tttttaaggt gagaaatagg aaagaaaacg 240

ccggcttgtg cgctcgctgc ctgcctctct ggctgtctgc ttttgcaggg ctgctgggag 300

tttttaagct ctgtgagaat cctgggagtt ggtgatgtca gactagttgg gtcatttgaa 360

ggttagcagc ccgggtaggg ttcaccgaaa gttcactcgc atatattagg caattcaatc 420

tttcattctg tgtgacagaa gtagtaggaa gtgagctgtt cagaggcagg agggtctatt 480

ctttgccaaa ggggggacca gaattccccc atgcgagctg tttgaggact gggatgccga 540

gaacgcgagc gatccgagca gggtttgtct gggcaccgtc ggggtaggat ccggaacgca 600

ttcggaaggc tttttgcaag catttacttg gaaggagaac ttgggatctt tctgggaacc 660

ccccgccccg gctggattgg ccgagcaagc ctggaaaatg gtaaatgatc atttggatca 720

attacaggct tttagctggc ttgtctgtca taattcatga ttcggggctg ggaaaaagac 780

caacagccta cgtgccaaaa aaggggcaga gtttgatgga gttgggtg 828

<210> 10
<211> 684
<212> DNA
<213> Homo sapiens

<400> 10
atctccgcgt ggtgctttgc ggtcgccgtc gttgtggccg tccggggtgg ggtgtgagga 60

ggggacgaag gagggaagga agggcaaggc ggggggggct ctgcgagagc gcgcccagcc 120

ccgccttcgg gccccacagt ccctgcaccc aggtttccat tgcgcggctc tcctcagctc 180

cttcccgccg cccagtctgg atcctggggg aggcgctgaa gtcggggccc gccctgtggc 240

cccgcccggc ccgcgcttgc tagcgcccaa agccagcgaa gcacgggccc aaccgggcca 300

tgtcggggga gcctgagctc attgagctgc gggagctggc acccgctggg cgcgctggga 360

agggccgcac ccggctggag cgtgccaacg cgctgcgcat cgcgcggggc accgcgtgca 420

accccacacg gcagctggtc cctggccgtg gccaccgctt ccagcccgcg gggcccgcca 480

cgcacacgtg gtgcgacctc tgtggcgact tcatctgggg cgtcgtgcgc aaaggcctgc 540

agtgcgcgcg tgagtagtgg ccccgcgcgc ctacgagagc ggaaggggca gccaaggggc 600

agcgcagtcg ccgcgggtca agtcgcggca gagggggtcg gcggggacag ctcccgagga 660

ctaggtccgt tactttcgcc ccat 684

<210> 11
<211> 896
<212> DNA
<213> Homo sapiens

<400> 11
ggcgtcaggc tcgcaaagaa cagtttgggg tttaagaaat cggcctagcg attcgcgcca 60

gcgcgcccgg ccgcctgggt ctgcgactcg gactcaccca gcatcttgct caactcggcg 120

ttgtgcaggt ctggattctg ctgcgccagc cgcttgcgct cgtccttagc ccacaccatg 180

aaagcgttca tcggccgccg gatacgggac tcgcccttgg ctcggcccgc ggccccggcc 240

ggtgctccgc tgttcgccgg cgcctcgccc ttcaccttca tgtccccgat ggggctcagc 300

gactcggccc aggggcaggg gcccagcccg gccatcaccg cgggcagcgc gctctgggtc 360

tggctctggt cgtcactggc gtatcccgca tccgggctgc tcatggcgct ccaggcctgc 420

cccgctcccc tcaaccctcc gccggacgcg ccgcctcccc cgacccgggg gagggggtgg 480

ggagtgaggc actgagatgc cccgagggct gcgcgggtct cccggcccga agccgccgcc 540

cgtgttctgg cctgtcgcgg tctggtctac agcgtaccca gggcccccag ccggcctagt 600

gacactgcgg gcgcccctgg gccgcggggc cttttctgca cagatgtggc caatggagcg 660

gcgagggcgg gccggtcccg cgtcgttagg cccacgccca ggccgtggtc cgagtcccac 720

gtcccagtcc aaccccacgc ccgccccttg cccctccccc ggccccgcgc ccaaggctac 780

acctgccccc gggaaaacta gccggagctg ggctctggcg ccgcgtcctc tcccaccggg 840

gttagggaga ctcgaaaagc cgtctgggag ggctgattgt accttggaat cgacgt 896

<210> 12
<211> 22
<212> DNA
<213> artificial

<220>
<223> primer

<400>  12
tcgcgagggt atattttcga gg                                                22

<210>  13
<211>  22
<212>  DNA
<213>  artificial

<220>
<223> primer

<400>  13
atccgcatcc aacgaattac ac                                                22

<210>  14
<211>  26
<212>  DNA
<213>  artificial

<220>
<223>  blocking primer

<400>  14
ttgaggggta tggggttagg gttagg                                            26

<210>  15
<211>  21
<212>  DNA
<213>  artificial

<220>
<223>  probe

<400>  15
acacaaaacc ccgcccaacc g                                                 21

<210>  16
<211>  22
<212>  DNA
<213>  artificial

<220>
<223>  primer

<400>  16
ggcgtttttt attttcgtcg gg                                                22

<210>  17
<211>  21
<212>  DNA
<213>  artificial

<220>
<223>  primer

<400>  17
taccccattt ccgaatccga c                                                 21

<210>  18
<211>  32
<212>  DNA
<213>  artificial

<220>
<223>  blocking primer

<400>  18
gttttttatt tttgttggga gtttgttgat tg                                    32


<210>  19
<211>  22
<212>  DNA
<213>  artificial

<220>
<223>  probe

<400>  19
tacgcccaca cccaaccaat cg                                              22


<210>  20
<211>  23
<212>  DNA
<213>  artificial

<220>
<223>  primer

<400>  20
tacgttattc gggggtagat tgg                                             23


<210>  21
<211>  19
<212>  DNA
<213>  artificial

<220>
<223>  primer

<400>  21
cgcgcttttc cgttaccac                                                  19


<210>  22
<211>  25
<212>  DNA
<213>  artificial

<220>
<223>  blocking primer

<400>  22
tgttatttgg gggtagattg ggtgg                                           25


<210>  23

<211>  32
<212>  DNA
<213>  artificial

<220>
<223>  probe

<400>  23
aaaccaaacg tctctcgaaa ctctaaatta ac                                    32


<210>  24
<211>  23
<212>  DNA
<213>  artificial

<220>
<223>  primer

<400>  24
acgtcggttg gttatgaggt tag                                              23


<210>  25
<211>  25
<212>  DNA
<213>  artificial

<220>
<223>  primer

<400>  25
cgaattctaa cgattctcct acctc                                            25


<210>  26
<211>  29
<212>  DNA
<213>  artificial

<220>
<223>  blocking primer

<400>  26
tgttggttgg ttatgaggtt aggagtttt                                        29


<210>  27
<211>  29
<212>  DNA
<213>  artificial

<220>
<223>  probe

<400>  27
aaacgaaatt tcaccacgtt aatcaaact                                        29


<210>  28
<211>  21
<212>  DNA
<213>  artificial

<220>
<223>  primer

<400>  28
gcgtgggaga gtggattttc g                                                21


<210>  29
<211>  19
<212>  DNA
<213>  artificial

<220>
<223>  primer

<400>  29
tcctcaacgc ttccctcgc                                                   19


<210>  30
<211>  30
<212>  DNA
<213>  artificial

<220>
<223>  blocking primer

<400>  30
tgtgggagag tggatttttg aagttgatag                                        30


<210>  31
<211>  21
<212>  DNA
<213>  artificial

<220>
<223>  probe

<400>  31
aaattccgcc cctaccccc g                                                 21


<210>  32
<211>  26
<212>  DNA
<213>  artificial

<220>
<223>  primer

<400>  32
tacggtttat ttagttcgcg ttttat                                           26


<210>  33
<211>  27
<212>  DNA
<213>  artificial

<220>
<223>  primer

<400>  33
ccaacgccct aatatactaa ccaaact                                          27


<210>  34
<211>  35
<212>  DNA
<213>  artificial

<220>
<223>  blocking primer

<400>  34
tttagtttgt gttttatttt gttttgttgg ttttt                                 35


<210>  35
<211>  25
<212>  DNA
<213>  artificial

<220>
<223>  probe

<400>  35
aaacccgaac aaaaacgcga aaaac                                            25


<210>  36
<211>  24
<212>  DNA
<213>  artificial

<220>
<223>  primer

<400>  36
tgcgttagag tacgtgttag ggtc                                             24


<210>  37
<211>  25
<212>  DNA
<213>  artificial

<220>
<223>  primer

<400>  37
cgaataaaaa attaaatccg actcc                                            25


<210>  38
<211>  29
<212>  DNA
<213>  artificial

<220>
<223>  blocking primer

<400>  38
gagtatgtgt tagggttgat tgtgttggt                                        29

<210>  39
<211>  25
<212>  DNA
<213>  artificial

<220>
<223>  probe

<400>  39
aaccgactac gataaaatcg ccgcc                                          25


<210>  40
<211>  26
<212>  DNA
<213>  artificial

<220>
<223>  primer

<400>  40
gttgtatcgg tgtggttacg tttagc                                         26


<210>  41
<211>  23
<212>  DNA
<213>  artificial

<220>
<223>  primer

<400>  41
tctaaaaact cttcgaacgc cgt                                            23


<210>  42
<211>  34
<212>  DNA
<213>  artificial

<220>
<223>  blocking primer

<400>  42
attggtgtgg ttatgtttag tgtagatatt ttgg                                34


<210>  43
<211>  27
<212>  DNA
<213>  artificial

<220>
<223>  probe

<400>  43
tcgcccctac atctactaac aaaccgc                                        27


<210>  44

<211>  19
<212>  DNA
<213>  artificial

<220>
<223>  primer

<400>  44
gaagcgtttt ggtacgcgg                                                    19


<210>  45
<211>  17
<212>  DNA
<213>  artificial

<220>
<223>  primer

<400>  45
atacgccccc gcgacct                                                      17


<210>  46
<211>  28
<212>  DNA
<213>  artificial

<220>
<223>  blocking primer

<400>  46
tgttttggta tgtgggtgtg tgttgttt                                          28


<210>  47
<211>  28
<212>  DNA
<213>  artificial

<220>
<223>  probe

<400>  47
tacgaaattc aaaaaaccca aaaaaccg                                          28


<210>  48
<211>  19
<212>  DNA
<213>  artificial

<220>
<223>  primer

<400>  48
tttttcgttg gcgttgggt                                                    19


<210>  49
<211>  23
<212>  DNA
<213>  artificial

<220>
<223>  primer

<400>  49
acacctacga atcgtcgaac gac                                              23


<210>  50
<211>  25
<212>  DNA
<213>  artificial

<220>
<223>  blocking primer

<400>  50
tgttggtgtt gggtttggtt ttggt                                            25


<210>  51
<211>  27
<212>  DNA
<213>  artificial

<220>
<223>  probe

<400>  51
tcgcgtaaca acgccaaaaa acaaaat                                          27


<210>  52
<211>  25
<212>  DNA
<213>  artificial

<220>
<223>  primer

<400>  52
tcgaagagta ttttcgggag ttttg                                            25


<210>  53
<211>  22
<212>  DNA
<213>  artificial

<220>
<223>  primer

<400>  53
ttaacgcttt cgaaaaacca cc                                               22


<210>  54
<211>  28
<212>  DNA
<213>  artificial

<220>
<223>  blocking primer

<400>  54
ttttgggagt tttgttagtg ggtttttg                                          28


<210>  55
<211>  30
<212>  DNA
<213>  artificial

<220>
<223>  probe

<400>  55
tacgctcttt cttcattcgc acaatctaac                                        30


<210>  56
<211>  24
<212>  DNA
<213>  artificial

<220>
<223>  primer

<400>  56
tagcgtattt ggaagatgcg tgat                                              24


<210>  57
<211>  25
<212>  DNA
<213>  artificial

<220>
<223>  primer

<400>  57
taaccgtcct acaacgcctc ataat                                             25


<210>  58
<211>  34
<212>  DNA
<213>  artificial

<220>
<223>  blocking primer

<400>  58
gatgtgtgat tttgttgtgt gatagtttta gggt                                   34


<210>  59
<211>  32
<212>  DNA
<213>  artificial

<220>
<223>  probe

<400>  59
   tacccccgac accttcaata acctaataaa ac                                    32

<210>  60
<211>  20
<212>  DNA
<213>  artificial

<220>
<223>  primer

<400>  60
ttggcggata ggggaagggt                                                    20


<210>  61
<211>  25
<212>  DNA
<213>  artificial

<220>
<223>  primer

<400>  61
acgcgaacgc tcctatatat taacc                                              25


<210>  62
<211>  25
<212>  DNA
<213>  artificial

<220>
<223>  blocking primer

<400>  62
tggatagggg aagggttggg tgtgt                                              25


<210>  63
<211>  32
<212>  DNA
<213>  artificial

<220>
<223>  probe

<400>  63
tctactatac gcgatactcg aacttctacg cg                                      32


<210>  64
<211>  18
<212>  DNA
<213>  artificial

<220>
<223>  primer

<400>  64
tttcggttag ttgcgcgg                                                      18


<210>  65

<211>  21
<212>  DNA
<213>  artificial

<220>
<223>  primer

<400>  65
ctaaaaaatc ccgcgaactc c                                                21


<210>  66
<211>  26
<212>  DNA
<213>  artificial

<220>
<223>  blocking primer

<400>  66
ttgtgtggtg attttgggga ttttag                                           26


<210>  67
<211>  24
<212>  DNA
<213>  artificial

<220>
<223>  probe

<400>  67
accgcaaaaa aacgccctaa aatc                                             24


<210>  68
<211>  21
<212>  DNA
<213>  artificial

<220>
<223>  primer

<400>  68
tcgttggagt ttcgtcgtcg t                                                21


<210>  69
<211>  18
<212>  DNA
<213>  artificial

<220>
<223>  primer

<400>  69
aacccccgtc ccgaatct                                                    18


<210>  70
<211>  35
<212>  DNA
<213>  artificial

<220>
<223>  blocking primer

<400>  70
ggagttttgt tgttgtagtt tttgttatta gtgag                                35


<210>  71
<211>  22
<212>  DNA
<213>  artificial

<220>
<223>  probe

<400>  71
acgcgaaccg cgcgtactca ct                                              22


<210>  72
<211>  22
<212>  DNA
<213>  artificial

<220>
<223>  primer

<400>  72
ttcgtgtggg gagtgacgtg ac                                              22


<210>  73
<211>  21
<212>  DNA
<213>  artificial

<220>
<223>  primer

<400>  73
tcgtttctcg tcgcccctac t                                               21


<210>  74
<211>  35
<212>  DNA
<213>  artificial

<220>
<223>  blocking primer

<400>  74
gtggggagtg atgtgatgtt agtagagatt ttatt                                35


<210>  75
<211>  21
<212>  DNA
<213>  artificial

<220>
<223>  probe

<400>  75
accgcccgcg cgccactata c                                                21

<210>  76
<211>  20
<212>  DNA
<213>  artificial

<220>
<223>  primer

<400>  76
cggagtcggg agagcgaaag                                                  20

<210>  77
<211>  28
<212>  DNA
<213>  artificial

<220>
<223>  primer

<400>  77
taaaactacc gaattaacgc aaattact                                         28

<210>  78
<211>  26
<212>  DNA
<213>  artificial

<220>
<223>  blocking primer

<400>  78
ttgggagagt gaaaggagag gggatt                                           26

<210>  79
<211>  28
<212>  DNA
<213>  artificial

<220>
<223>  probe

<400>  79
tcctcgatta actcctaaat accccgcc                                         28

<210>  80
<211>  23
<212>  DNA
<213>  artificial

<220>
<223>  primer

<400>  80
tgaggagacg gtcgatagtt tgg                                              23

<210>  81
<211>  25
<212>  DNA
<213>  artificial

<220>
<223>  primer

<400>  81
acgaacaacg acgactacga aaact                                          25


<210>  82
<211>  32
<212>  DNA
<213>  artificial

<220>
<223>  blocking primer

<400>  82
gagatggttg atagtttggt ttttgttgtt tg                                   32


<210>  83
<211>  28
<212>  DNA
<213>  artificial

<220>
<223>  probe

<400>  83
taaaaaaccc gcttcactta aaacaccg                                       28


<210>  84
<211>  24
<212>  DNA
<213>  artificial

<220>
<223>  primer

<400>  84
gagacggaaa gttagcgggt aaac                                           24


<210>  85
<211>  22
<212>  DNA
<213>  artificial

<220>
<223>  primer

<400>  85
caaaacgcga tcgacgttaa ct                                             22


<210>  86

<211>  31
<212>  DNA
<213>  artificial

<220>
<223>  blocking primer

<400>  86
atggaaagtt agtgggtaaa tgaagatatg g                                    31


<210>  87
<211>  34
<212>  DNA
<213>  artificial

<220>
<223>  probe

<400>  87
tactccctaa aaacgctcta aataataccc ttct                                 34


<210>  88
<211>  25
<212>  DNA
<213>  artificial

<220>
<223>  primer

<400>  88
gcgtatagag gaatttaaag tgtgg                                           25


<210>  89
<211>  27
<212>  DNA
<213>  artificial

<220>
<223>  primer

<400>  89
acgccttttt atttacgacg acttaac                                         27


<210>  90
<211>  39
<212>  DNA
<213>  artificial

<220>
<223>  blocking primer

<400>  90
ttatttacaa caacttaact taaaaaacaa tattccacc                            39


<210>  91
<211>  21
<212>  DNA
<213>  artificial

<220>
<223>  probe

<400>  91
tattccgcct acgcccgctc g                                                21


<210>  92
<211>  27
<212>  DNA
<213>  artificial

<220>
<223>  primer

<400>  92
   gaattttttt atgcgagttg tttgagg                                           27


<210>  93
<211>  25
<212>  DNA
<213>  artificial

<220>
<223>  primer

<400>  93
ttccgaatac gttccgaatc ctacc                                            25


<210>  94
<211>  33
<212>  DNA
<213>  artificial

<220>
<223>  blocking primer

<400>  94
ttatgtgagt tgtttgagga ttgggatgtt gag                                   33


<210>  95
<211>  26
<212>  DNA
<213>  artificial

<220>
<223>  probe

<400>  95
aacaaaccct actcgaatcg ctcgcg                                           26


<210>  96
<211>  21
<212>  DNA
<213>  artificial

<220>
<223>  primer

<400>  96
tgggaatttt tcgtttcggt t                                                21

<210>  97
<211>  29
<212>  DNA
<213>  artificial

<220>
<223>  primer

<400>  97
acacgtaaac tattaatctt tttcccaac                                        29

<210>  98
<211>  34
<212>  DNA
<213>  artificial

<220>
<223>  blocking primer

<400>  98
cataaactat taatcttttt cccaacccca aatc                                  34

<210>  99
<211>  30
<212>  DNA
<213>  artificial

<220>
<223>  probe

<400>  99
tcatttacca ttttccaaac ttactcgacc                                       30

<210>  100
<211>  16
<212>  DNA
<213>  artificial

<220>
<223>  primer

<400>  100
aaggaagggt aaggcg                                                      16

<210>  101
<211>  18
<212>  DNA
<213>  artificial

<220>
<223>  primer

<400>  101
cgaacgaaac cacaaaac                                                    18

<210>  102
<211>  19
<212>  DNA
<213>  artificial

<220>
<223>  blocking primer

<400>  102
gtgggggggg ttttgtgag                                                    19


<210>  103
<211>  20
<212>  DNA
<213>  artificial

<220>
<223>  probe

<400>  103
aaccccgact tcaacgcctc                                                   20


<210>  104
<211>  21
<212>  DNA
<213>  artificial

<220>
<223>  primer

<400>  104
ttgttagcgt ttaaagttag c                                                 21


<210>  105
<211>  20
<212>  DNA
<213>  artificial

<220>
<223>  primer

<400>  105
taaaattaca cgcgataccc                                                   20


<210>  106
<211>  28
<212>  DNA
<213>  artificial

<220>
<223>  blocking primer

<400>  106
agtgtttaaa gttagtgaag tatgggtt                                          28


<210>  107

<211>  23
<212>  DNA
<213>  artificial

<220>
<223>  probe

<400>  107
acacgctcca accgaatacg acc                                               23


<210>  108
<211>  17
<212>  DNA
<213>  artificial

<220>
<223>  primer

<400>  108
tcgtttttag ttcgcgg                                                      17


<210>  109
<211>  18
<212>  DNA
<213>  artificial

<220>
<223>  primer

<400>  109
ttaactaccc cttccgct                                                     18


<210>  110
<211>  28
<212>  DNA
<213>  artificial

<220>
<223>  blocking primer

<400>  110
tagtttgtgg ggtttgttat gtatatgt                                          28


<210>  111
<211>  23
<212>  DNA
<213>  artificial

<220>
<223>  probe

<400>  111
acaaaccttt acgcacgacg ccc                                               23


<210>  112
<211>  19
<212>  DNA
<213>  artificial

<220>
<223>  primer

<400>  112
ttcggtcgtt tgggtttgc                                                    19


<210>  113
<211>  21
<212>  DNA
<213>  artificial

<220>
<223>  primer

<400>  113
caaaaacgaa tcccgtatcc g                                                 21


<210>  114
<211>  31
<212>  DNA
<213>  artificial

<220>
<223>  blocking primer

<400>  114
ttgtttgggt ttgtgatttg gatttattta g                                      31


<210>  115
<211>  28
<212>  DNA
<213>  artificial

<220>
<223>  probe

<400>  115
tccaaaccta cacaacgccg aattaaac                                          28


<210>  116
<211>  23
<212>  DNA
<213>  artificial

<220>
<223>  primer

<400>  116
ttagttcggt tattatcgcg ggt                                               23


<210>  117
<211>  19
<212>  DNA
<213>  artificial

<220>
<223>  primer

<400>  117
cgcgcaaccc tcgaaacat                                                    19


<210>  118
<211>  31
<212>  DNA
<213>  artificial

<220>
<223>  blocking primer

<400>  118
tggttattat tgtgggtagt gtgttttggg t                                      31


<210>  119
<211>  27
<212>  DNA
<213>  artificial

<220>
<223>  probe

<400>  119
tacgccaata acgaccaaaa ccaaacc                                           27


<210>  120
<211>  21
<212>  DNA
<213>  artificial

<220>
<223>  primer

<400>  120
tcgcggtttg gtttatagcg t                                                 21


<210>  121
<211>  25
<212>  DNA
<213>  artificial

<220>
<223>  primer

<400>  121
taaaactcga accacgacct aaacg                                             25


<210>  122
<211>  36
<212>  DNA
<213>  artificial

<220>
<223>  blocking primer

<400>  122
tggtttggtt tatagtgtat ttagggtttt tagttg                                 36

<210>  123
<211>  25
<212>  DNA
<213>  artificial

<220>
<223>  probe

<400>  123
cgcccgcaat atcactaaac cgact                                          25


<210>  124
<211>  23
<212>  DNA
<213>  artificial

<220>
<223>  primer

<400>  124
gtgatggagg aggtttagta agt                                            23


<210>  125
<211>  23
<212>  DNA
<213>  artificial

<220>
<223>  primer

<400>  125
ccaataaaac ctactcctcc ctt                                            23


<210>  126
<211>  30
<212>  DNA
<213>  artificial

<220>
<223>  probe

<400>  126
accaccaccc aacacacaat aacaaacaca                                     30